# EUROPÄISCHE PATENTSCHRIFT

(11) **EP 2 692 729 B1**
(45) Veröffentlichungstag und Bekanntmachung des Hinweises auf die Patenterteilung: **07.12.2016**
(21) Anmeldenummer: 13176159.5
(22) Anmeldetag: 11.07.2013
(51) Int. Cl.: C07H 17/07, C12N 9/90

(54) **Verfahren zur biotechnologischen Herstellung von Dihydrochalkonen**
Method for the biotechnological manufacture of dihydrochalcones
Procédé de fabrication biotechnologique de dihydrochalcones

(30) Priorität: 31.07.2012 DE 102012213492
(43) Veröffentlichungstag der Anmeldung: 05.02.2014
(73) Patentinhaber: Symrise AG, 37603 Holzminden (DE)
(72) Erfinder: Hilmer, Dr. Jens-Michael, 37603 Holzminden (DE); Gross, Egon, 37603 Holzminden (DE); Krammer, Dr. Gerhard, 37603 Holzminden (DE); Ley, Dr. Jakob Peter, 37603 Holzminden (DE); Gall, Mechthild, 17489 Greifswald (DE); Bornscheuer, Prof. Dr. rer. nat. Uwe, 17489 Greifswald (DE); Thomsen, Maren, 17489 Greifswald (DE); Peters, Christin, 17489 Greifswald (DE); Jonczyk, Patrick, 30419 Hannover (DE); Beutel, Dr. rer. nat. Sascha, 30169 Hannover (DE); Scheper, Prof. Dr. rer. nat. Tomas, 30559 Hannover (DE)
(74) Vertreter: Eisenführ Speiser

(56) Entgegenhaltungen:
- US-A1- 2005 208 643
- SCHMIDT A ET AL: "Biocatalytic formation of a bioactive dihydrochalcone by Eubacterium ramulus", JOURNAL OF BIOTECHNOLOGY, ELSEVIER SCIENCE PUBLISHERS, AMSTERDAM, NL, Bd. 150, 1. November 2010 (2010-11-01), Seite 307, XP027489759, ISSN: 0168-1656, DOI: 10.1016/J.JBIOTEC.2010.09.276 [gefunden am 2010-11-01]
- SCHNEIDER H ET AL: "Anaerobic degradation of flavonoids by Eubacterium ramulus", ARCHIVES OF MICROBIOLOGY, SPRINGER, DE, Bd. 173, Nr. 1, 1. Januar 2000 (2000-01-01), Seiten 71-75, XP008115183, ISSN: 0302-8933, DOI: 10.1007/S002030050010
- HERLES CLAUDIA ET AL: "First bacterial chalcone isomerase isolated from Eubacterium ramulus", ARCHIVES OF MICROBIOLOGY, Bd. 181, Nr. 6, Juni 2004 (2004-06), Seiten 428-434, XP002713878, ISSN: 0302-8933
- HWANG E I ET AL: "Production of plant-specific flavanones by Escherichia coli containing an artificial gene cluster", APPLIED AND ENVIRONMENTAL MICROBIOLOGY, AMERICAN SOCIETY FOR MICROBIOLOGY, US, Bd. 69, Nr. 5, 1. Mai 2003 (2003-05-01), Seiten 2699-2706, XP008117399, ISSN: 0099-2240, DOI: 10.1128/AEM.69.5.2699-2706.2003
- DATABASE UniProt [Online] 1. Februar 1995 (1995-02-01), "RecName: Full=Chalcone--flavonone isomerase 1; Short=Chalcone isomerase 1; EC=5.5.1.6; AltName: Full=Protein TRANSPARENT TESTA 5;", XP002713879, gefunden im EBI accession no. UNIPROT:P41088 Database accession no. P41088

## Beschreibung

Die vorliegende Erfindung betrifft primär ein Verfahren zur Herstellung eines Dihydrochalkons, insbesondere von Phloretin, bzw. ein Verfahren zur Reduktion von Flavanonen unter Verwendung eines transgenen Mikroorganismus, enthaltend einen Nukleinsäure-Abschnitt (a), umfassend oder bestehend aus einem für eine bakterielle Chalconisomerase kodierenden Gen, und/oder einen Nukleinsäure-Abschnitt (a'), umfassend oder bestehend aus einem für eine pflanzliche Chalconisomerase kodierenden Gen, sowie einen Nukleinsäure-Abschnitt (b), umfassend oder bestehend aus einem für eine bakterielle Enoat-Reduktase kodierenden Gen.

Weiterhin betrifft die vorliegende Erfindung einen transgenen Mikroorganismus, der einen Nukleinsäure-Abschnitt (a), umfassend oder bestehend aus einem für eine bakterielle Chalconisomerase kodierenden Gen, und/oder einen Nukleinsäure-Abschnitt (a'), umfassend oder bestehend aus einem für eine pflanzliche Chalconisomerase kodierenden Gen, sowie einen Nukleinsäure-Abschnitt (b), umfassend oder bestehend aus einem für eine bakterielle Enoat-Reduktase kodierenden Gen, enthält.

Die vorliegende Erfindung betrifft auch einen Vektor, insbesondere einen Plasmidvektor, enthaltend einen Nukleinsäure-Abschnitt (a), umfassend oder bestehend aus einem für eine bakterielle Chalconisomerase kodierenden Gen, und/oder einen Nukleinsäure-Abschnitt (a'), umfassend oder bestehend aus einem für eine pflanzliche Chalconisomerase kodierenden Gen, und/oder einen Nukleinsäure-Abschnitt (b), umfassend oder bestehend aus einem für eine bakterielle Enoat-Reduktase kodierenden Gen.

Zudem betrifft die vorliegende Erfindung eine Wirtszelle, die ein oder mehrere identische oder unterschiedliche erfindungsgemäße Vektoren enthält.

Weitere Aspekte der vorliegenden Erfindung ergeben sich aus der nachfolgenden Beschreibung, den Beispielen sowie insbesondere den beigefügten Patentansprüchen.

Dihydrochalcone, insbesondere Phloretin, werden üblicherweise entweder durch chemische Reduktion von Chalkonen oder durch Friedel-Crafts-artige Acylierung von Phenolen mit Dihydrozimtsäuren hergestellt. Nachteil dieser Verfahren ist, dass damit hergestellte Lebensmittelzusatzstoffe, Geschmackstoffe oder Aromastoffe nicht als natürlich bezeichnet werden dürfen. Zudem können Dihydrochalcone, insbesondere Phloretin, durch Extraktion z.B. des entsprechenden Glycosids (z. B. aus Malus spp.-Rohstoffen) mit anschließender Generierung des Aglycons gewonnen werden. Dieser Prozess ist jedoch zeit- und kostenaufwändig und zudem Saison-abhängig.

Bedeutende Geschmacks- und Aromastoffe mit Dihydrochalkonstruktur sind z.B. Phloretin (z.B. nach EP 1,998,636-B1), Phloridzin, Trilobatin (vgl. Tanaka, T.; Yamasaki, K.; Kohda, H.; Tanaka, O.; Mahato, S. B., Dihydrochalcone-glucosides as sweet principles of Symplocos ssp. Planta Medica 1980, (Suppl.), 81-83), Naringindihydrochalkon und Neohesperidindihydrochalkon (Crosby, G. A., New Sweeteners. Critical Reviews in Food Science and Nutrition 1976, (June), 297-323).

Aufgrund des bestehenden und auch zukünftigen Bedarfs an vorteilhaften Dihydrochalkonen, insbesondere an Phloretin, bestand die primäre Aufgabe der vorliegenden Erfindung darin, ein effizientes, in industriellem Maßstab durchführbares und vorzugsweise kostengünstiges Verfahren zur Herstellung von Dihydrochalkonen, insbesondere von Phloretin, bereitzustellen.

Eine weitere Aufgabe bestand in der Bereitstellung geeigneter bzw. notwendiger Mittel, um ein solches Verfahren durchzuführen.

Weitere Aufgaben der vorliegenden Erfindung ergeben sich aus der nachfolgenden Beschreibung sowie insbesondere den beigefügten Patentansprüchen.

Die primäre Aufgabe der vorliegenden Erfindung wird gelöst durch ein innovatives biotechnologisches Verfahren zur Herstellung eines Dihydrochalkons, insbesondere von Phloretin, unter Verwendung eines transgenen Mikroorganismus, umfassend folgende Schritte:
(i) Bereitstellen eines transgenen Mikroorganismus, enthaltend (als Transgene)
   - einen Nukleinsäure-Abschnitt (a), umfassend oder bestehend aus einem für eine bakterielle Chalconisomerase kodierenden Gen,
      und/oder einen Nukleinsäure-Abschnitt (a'), umfassend oder bestehend aus einem für eine pflanzliche Chalconisomerase kodierenden Gen, sowie
   - einen Nukleinsäure-Abschnitt (b), umfassend oder bestehend aus einem für eine bakterielle Enoat-Reduktase kodierenden Gen,
(ii) Hinzufügen von einem oder mehreren Flavanonen, insbesondere Hinzufügen von Naringin, und/oder von einer oder mehreren Vorstufen oder einem oder mehreren Derivaten davon, insbesondere von einer Vorstufe oder einem Derivat von Naringin, zu dem transgenen Mikroorganismus und Kultivieren des transgenen Mikroorganismus unter Bedingungen, die die Umsetzung des bzw. der Flavanone und/oder der Vorstufe(n) oder des Derivats bzw. der Derivate davon, insbesondere von Naringin und/oder der Vorstufe bzw. des Derivats von Naringin, zu einem Dihydrochalkon, insbesondere zu Phloretin, ermöglichen,
(iii) optional: Isolieren sowie gegebenenfalls Aufreinigen des Dihydrochalkons, insbesondere von Phloretin,
wobei der transgene Mikroorganismus kein Mikroorganismus der Ordnung Clostridiales ist und wobei der Mikroorganismus Chalconisomerase- und Enoat-Reduktase-Aktivität aufweist, jedoch keine Phloretinhydrolase-Aktivität.

Das bzw. ein, mehrere oder sämtliche der erfindungsgemäß zu verwendenden Flavanone bzw. Vorstufen oder Derivate davon sind vorzugsweise ausgewählt aus der Gruppe bestehend aus:
Naringenin, Naringin, Narirutin, oder andere Naringeninglycoside, Hesperetin, Hesperidin, Neohesperidin, Hesperetin-7-O-glucosid, oder andere Hesperetinglycoside, Eriodictyol, Eriocitrin, oder andere Eriodictyolglycoside, Sterubin, Sterubinglycoside, Sakuranetin, Sakuranetinglycoside, Isosakuranetin, Isosakuranetinglycoside, 4',7-Dihydroxy-flavanon oder dessen Glycoside, 4',7-Dihydroxy-3'-methoxy-flavanon oder dessen Glycoside, 3',7-Dihydroxy-4'-methoxy-flavanon oder dessen Glycoside, 3',4',7-Trihydroxy-flavanon oder dessen Glycoside, wobei die Flavanone bezüglich der 2-Position des Flavanongerüsts als (S)-, als (R)-Enantiomer, als Racemat oder als beliebige Mischung der beiden Enantiomere vorliegen können.

Im Folgenden sind einige der bevorzugt einzusetzenden Flavanone exemplarisch abgebildet:

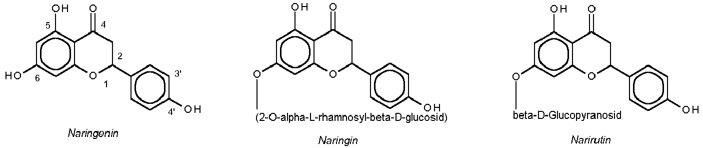

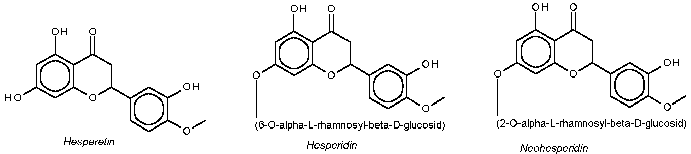

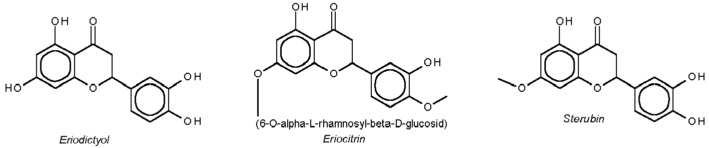

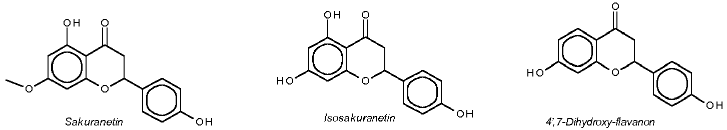

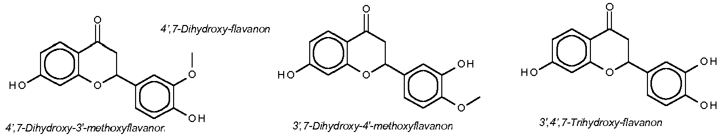

Das erfindungsgemäß herzustellende Dihydrochalkon ist vorzugsweise ausgewählt aus der Gruppe bestehend aus:
Phloretin, Naringindihydrochalkon, Phloridzin oder andere Phloretin-Glycoside , Hesperetindihydrochalkon, Hesperidindihydrochalkon, Neohesperidindihydrochalkon, oder andere Hesperetindihydrochalkonglycoside, Eriodictyoldihydrochalkon (3-Hydroxyphloretin), oder andere Eriodictyoldihydrochalkonglycoside, Sterubindihydrochalkon, Sterubindihydrochalkonglycoside, Sakuranetindihydrochalkon, Sakuranetindihydrochalkonglycoside, Isosakuranetindihydrochalkon, Isosakuranetindihydrochalkonglycoside, 2',4',4-Trihydroxydihydrochalkon (Davidigenin) oder dessen Glycoside, 3-Methoxy-2',4',4-trihydroxydihydrochalkon oder dessen Glycoside, 4-Methoxy-2',3,4'-trihydroxydihydrochalkon oder dessen Glycoside oder 2',4,4',3-Tetrahydroxydihydrochalkon oder dessen Glycoside.

Im Folgenden sind erfindungsgemäß bevorzugte Dihydrochalkone abgebildet:

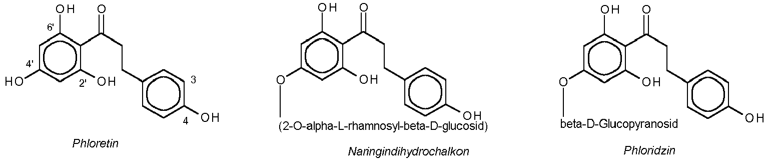

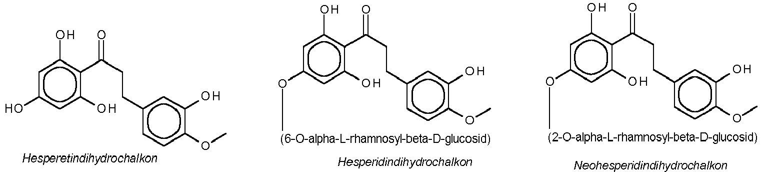

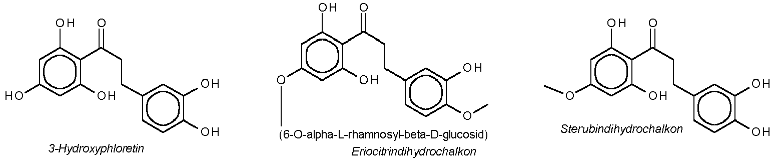

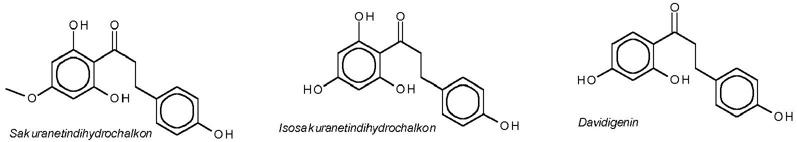

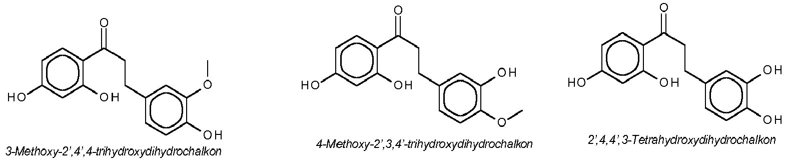

Besonders bevorzugte Flavanone und die jeweils daraus gebildeten Dihydrochalkone sind: Naringenin und Phloretin, Naringin und Naringindihydrochalkon, Narirutin und Phloridzin, Hesperetin und Hesperetindihydrochalkon, Hesperidin und Hesperidindihydrochalkon, Neohesperidin und Neohesperidindihydrochalkon sowie Eriodictyol und 3-Hydroxyphloretin.

Ein Aspekt der vorliegenden Erfindung betrifft demnach ein biotechnologisches Verfahren zur Produktion von (natürlichen) Dihydrochalkonen, insbesondere von (natürlichem) Phloretin, ausgehend von einem oder mehreren entsprechenden Flavanonen und/oder einer Vorstufe bzw. eines Derivats davon, insbesondere von Naringin und/oder einer Vorstufe bzw. eines Derivats von Naringin, insbesondere von Naringin oder Naringenin, unter Verwendung einer bakteriellen Chalconisomerase (besonders bevorzugt aus Mikroorganismen wie weiter unten beschrieben) und/oder einer pflanzlichen Chalconisomerase (besonders bevorzugt aus Pflanzen wie weiter unten beschrieben) in Kombination mit einer bakteriellen Enoat-Reduktase (bevorzugt aus Mikroorganismen wie weiter unten beschrieben) in einem transgenen Mikroorganismus.

Eine bevorzugte Ausführungsform betrifft ein biotechnologisches Verfahren zur Produktion von (natürlichem) Phloretin, ausgehend von Naringin und/oder einer Vorstufe bzw. eines Derivats von Naringin, insbesondere von Naringin, Narirutin oder Naringenin, unter Verwendung einer oder mehrerer Chalconisomerasen (wie hierin beschrieben) in Kombination mit einer Enoat-Reduktase (wie hierin beschrieben) unter Verwendung eines transgenen Mikroorganismus (wie ebenfalls hierin beschrieben).

Eine besonders bevorzugte Ausführungsform betrifft ein biotechnologisches Verfahren zur Produktion von (natürlichem) Phloretin, ausgehend von Naringin und/oder einer Vorstufe bzw. eines Derivats von Naringin, insbesondere von Naringin, Narirutin oder Naringenin, unter Verwendung einer bakteriellen Chalconisomerase (bevorzugt aus dem anaeroben Organismus *Eubacterium ramulus,* wie weiter unten beschrieben) in Kombination mit einer pflanzlichen Chalconisomerase (bevorzugt aus *Arabidopsis thaliana* oder *Medicago sativa,* wie weiter unten beschrieben) sowie einer bakteriellen Enoat-Reduktase (ebenfalls bevorzugt aus dem anaeroben Organismus *Eubacterium ramulus,* wie weiter unten beschrieben) unter Verwendung eines transgenen Mikroorganismus (wie hierin beschrieben).

Im Stand der Technik war bekannt, dass der anaerobe Mikroorganismus *Eubacterium ramulus* in der Lage ist, Naringenin abzubauen, wobei intermediär Phloretin gebildet wird. Hierunter ist jedoch kein (biotechnologisches) Verfahren zur Herstellung von Phloretin im Sinne der vorliegenden Erfindung zu verstehen, insbesondere kein Verfahren (wie oben beschrieben), das sich zur industriellen Herstellung von Phloretin eignet, d.h. zur Herstellung von Phloretin im industriellen Maßstab. Das intermediär gebildete Phloretin wird in *Eubacterium ramulus* nämlich unmittelbar weiter verstoffwechselt (vgl. Schneider et al, Anaerobic degradation of flavonoids by Eubacterium ramulus, Arch Microbiol (2000) 173 : 71-75), wie in dem folgenden Reaktionsschema dargestellt ist (siehe insbesondere die durch die Phloretinhydrolase (PhH) vermittelte Reaktion):

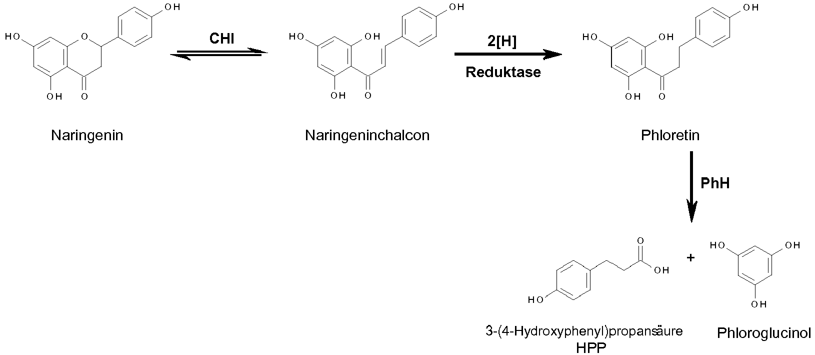

Im Rahmen der Untersuchungen im Zusammenhang mit der vorliegenden Erfindung konnten für die Zwecke des hierin beschriebenen Verfahrens entscheidende molekularbiologische und biochemische Grundlagen der Biotransformation - mit dem Ziel der Produktion von Phloretin in industriellem Maßstab (ohne unmittelbaren Abbau des gebildeten Phloretin) - aufgeklärt und charakterisiert werden.

Im Stand der Technik sind zwar unterschiedliche Einsatzmöglichkeiten von Enzymsystemen und Methoden der mikrobiellen Biotransformation beschrieben; bekannt ist beispielsweise die Möglichkeit einer einfachen Reduktion von Doppelbindungen mittels Hefen (z.B. Saccharomyces). Enzymatische Etherspaltungen wurden bisher jedoch kaum untersucht.

Im Zusammenhang mit der vorliegenden Erfindung sei grundsätzlich auf folgende Veröffentlichungen verwiesen: Schoefer et al, Anaerobic Degradation of Flavonoids by Clostridium orbiscindens, Appl. Environ. Microbiol., Oct. 2003, p. 5849-5854; und Herles et al, First bacterial chalcone isomerase isolated from Eubacterium ramulus, Arch Microbiol (2004) 181 : 428-434. Erkenntnisse im Zusammenhang mit dem Abbau von Lignin sind beispielsweise von Masai et al. beschrieben worden (Masai et al., 1993; Otsuka et al., 2003; s. auch JP 2002034557).

In WO 2006010117 von Koffas et al. und WO 2005084305 von Schmidt-Dannert et al. ist die Anwendung heterologer Expression zur Bildung von Flavonoiden beschrieben. Darin werden (ausschließlich) pflanzliche Gene beschrieben, die für eine heterologe Expression verschiedener Substanzen (ausgehend von L-Phenylalanin, Tyrosin und Zimtsäure) genutzt werden.

Zudem sei an dieser Stelle auf folgende weitere Dokumente des Standes der Technik verwiesen, die das selbe oder ein ähnliches technisches Gebiet betreffen:
SCHMIDT A ET AL: JOURNAL OF BIOTECHNOLOGY, ELSEVIER SCIENCE PUBLISHERS, AMSTERDAM, NL, Bd. 150, 1. November 2010, Seite 307, XP027489759
SCHNEIDER H ET AL: ARCHIVES OF MICROBIOLOGY, SPRINGER, DE, Bd. 173, Nr. 1,1. Januar 2000, Seiten 71 -75, XP008115183
HERLES CLAUDIA ET AL: ARCHIVES OF MICROBIOLOGY, Bd. 181, Nr. 6, Juni 2004 (2004-06), Seiten 428-434
HWANG E I ET AL: APPLIED AND ENVIRONMENTAL MICROBIOLOGY, AMERICAN SOCIETY FOR MICROBIOLOGY, US, Bd. 69, Nr. 5, 1. Mai 2003, Seiten 2699-2706, XP008117399

US 2005/208643 A1 (SCHMIDT-DANNERT CLAUDIA [US] ET AL) 22. September 2005

Überraschenderweise konnten anhand der Genomsequenz von *Eubacterium ramulus* die Gene kodierend (a) für eine Chalconisomerase und (b) für eine Enoatreduktase (zur Umsetzung von Naringin bzw. einer Vorstufe oder eines Derivats von Naringin zu Phloretin; vgl. hierzu das oben gezeigte Reaktionsschema) identifiziert und daraufhin heterolog in transgenen Mikroorganismen exprimiert werden. Durch die heterologe Expression dieser Enzyme in einem transgenen Mikroorganismus gelingt es vorteilhafterweise, die in *E. ramulus* üblicherweise stattfindende Folgereaktion von Phloretin zu Phloroglucinol und 3-(4-Hydroxyphenyl)propansäure (HPP) durch die Phloretinhydrolase (vgl. hierzu das oben gezeigte Reaktionsschema) zu vermeiden bzw. zu umgehen, um im Ergebnis eine Herstellung von Phloretin in industriellem Maßstab bzw. eine signifikant erhöhte Produktausbeute zu ermöglichen.

Unter einem "transgenen Mikroorganismus" ist im Zusammenhang mit der vorliegenden Erfindung ein gentechnisch veränderter bzw. modifizierter Mikroorganismus zu verstehen, in den gezielt mittels biotechnologischer Verfahren Nukleinsäure-Abschnitte (siehe Nukleinsäure-Abschnitte (a) und (b) wie hierin beschrieben) bzw. Gene eines anderen Organismus (sog. Transgene) eingeschleust worden sind.

Eine "Chalconisomerase" (CHI) im Sinne der vorliegenden Erfindung ist ein Enzym, das die Reaktion "Chalcon 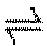 Flavanon" katalysiert. Insbesondere katalysiert die CHI die Reaktion von/zu Naringenin zu/von Naringeninchalcon (vgl. das oben gezeigte Reaktionsschema), für die Zwecke der vorliegenden Erfindung insbesondere die Reaktion von Naringenin zu Naringeninchalcon.

Eine "Enoat-Reduktase" (ERED) im Sinne der vorliegenden Erfindung ist ein Enzym, das die Dehydrierung bestimmter Verbindungen katalysiert, insbesondere die Reaktion von Naringeninchalcon zu Phloretin (vgl. das oben gezeigte Reaktionsschema).

In Anbetracht der oben erläuterten Zusammenhänge ist der im Rahmen des erfindungsgemäßen Verfahrens eingesetzte transgene Mikroorganismus nicht *Eubacterium ramulus,* und kein Mikroorganismus der Ordnung Clostridiales, bevorzugt kein Mikroorganismus der Klasse Clostridia, besonders bevorzugt kein Mikroorganismus des Phylus (Abteilung) der Firmicutes. Vielmehr ist der Mikroorganismus bevorzugt ausgewählt aus der Gruppe bestehend aus fakultativ anaeroben Mikroorganismen, insbesondere fakultativ aeroben Bakterien, bevorzugt Proteobakterien, insbesondere Enterobakterien, zum Beispiel der Gattung *Escherichia,* bevorzugt *E. coli,* insbesondere *E. coli* BL21, *E. coli* Rosetta (Derivat von *E. coli* BL 21) und *E. coli* SE1, und Hefen, zum Beispiel S. *cerevesiae* und *P. pastoris.* Gemäß einem bevorzugten Aspekt sind für die Zwecke der hierin beschriebenen Erfindung grundsätzlich solche Mikroorganismen bevorzugt, die unter aeroben Bedingungen wachsen und (auch) unter Sauerstoff-Ausschluss die eingeschleusten Gene (Transgene; s. oben) exprimieren können.

Erfindungsgemäß bevorzugt ist ein Verfahren (wie oben beschrieben), wobei in Schritt (ii) Naringin und/oder ein Aglycon davon hinzugefügt wird bzw. werden.

Vorzugsweise kodiert bzw. kodieren das für eine bakterielle Chalconisomerase kodierende Gen für eine Chalconisomerase aus einem Mikroorganismus aus dem Phylus der Firmicutes, vorzugsweise der Klasse Clostridia, insbesondere der Ordnung Clostridiales, besonders bevorzugt für eine Chalconisomerase aus *E. ramulus,*
und/oder
das für eine pflanzliche Chalconisomerase kodierende Gen für eine Chalconisomerase aus einer Pflanze der Ordnung der Brassicales, vorzugsweise der Familie der Brassicaceae, bevorzugt dem Tribus der Camelineae, insbesodnere der Gattung der Arabidopsis, vor allem der Art *Arabidopsis thaliana,* oder der Ordnung der Fabales, vorzugsweise der Familie der Fabaceae, bevorzugt der Unterfamilie der Faboidae, insbesondere der Gattung der Medicago, vor allem der Art *Medicago sativa,* also besonders bevorzugt für eine Chalconisomerase aus *A. thaliana* oder M. *sativa* (vgl. hierzu WO2005084305 A2 (Schmidt-Dannert) und WO2006010117A2 (Koffas)),
und/oder
das für eine bakterielle Enoat-Reduktase kodierende Gen für eine Enoat-Reduktase aus einem Mikroorganismus aus dem Phylus der Firmicutes, vorzugsweise der Klasse Clostridia, insbesondere der Ordnung Clostridiales, besonders bevorzugt für eine Enoat-Reduktase aus *E. ramulus.*

Besonders bevorzugt ist ein erfindungsgemäßes Verfahren (wie hierin beschrieben), wobei
der Nukleinsäure-Abschnitt (a) eine Nukleotidsequenz gemäß SEQ ID NO:**1** (Nukleotidsequenz des für die bakterielle CHI aus *E. ramulus* DSM 16296 kodierenden Gens) oder eine Nukleotidsequenz mit einer Nukleotidsequenz-Identität von 40 % oder mehr zu SEQ ID NO:**1**, vorzugsweise von 50 % oder mehr, 60 % oder mehr oder 80 % oder mehr, besonders bevorzugt von 95 % oder mehr, umfasst oder daraus besteht
und/oder
der Nukleinsäure-Abschnitt (a') eine Nukleotidsequenz gemäß SEQ ID NO:**6** (Nukleotidsequenz der CHI aus M. *sativa* (cultivar Iroquois) (MsCHI-1) mRNA, complete cds) oder SEQ ID NO:7 (Nukleotidsequenz der chalcone-flavonone isomerase 1 (TT5) mRNA aus *Arabidopsis thaliana,* complete cds) oder eine Nukleotidsequenz mit einer Nukleotidsequenz-Identität von 40 % oder mehr zu SEQ ID NO:**6** oder SEQ ID NO:**7**, vorzugsweise von 50 % oder mehr, 60 % oder mehr oder 80 % oder mehr, besonders bevorzugt von 95 % oder mehr, umfasst oder daraus besteht
und/oder
der Nukleinsäure-Abschnitt (b) eine Nukleotidsequenz gemäß SEQ ID NO:**2** (Nukleotidsequenz des für die bakterielle ERED aus *E. ramulus* DSM 16296 kodierenden Gens) oder SEQ ID NO:**5** (codon optimierte Nukleotidsequenz des für die bakterielle ERED aus *E. ramulus* DSM 16296 kodierenden Gens, insbesondere zur Expression in *E. coli* BL21, integriert in pET 22b (kloniert mittels Nde1 und BamH1)) oder eine Nukleotidsequenz mit einer Nukleotidsequenz-Identität von 40 % oder mehr zu SEQ ID NO:**2** oder SEQ ID NO:**5**, vorzugsweise von 50 % oder mehr, 60 % oder mehr oder 80 % oder mehr, besonders bevorzugt von 95 % oder mehr, umfasst oder daraus besteht.

Erfindungsgemäß bevorzugt ist weiterhin ein Verfahren (wie oben beschrieben), wobei
die bakterielle Chalconisomerase eine Aminosäuresequenz gemäß SEQ ID NO:**3** (Aminosäuresequenz der bakteriellen CHI aus *E. ramulus* DSM 16296) oder eine Aminosäuresequenz mit einer Aminosäuresequenz-Identität von 40 % oder mehr zu SEQ ID NO:**3**, vorzugsweise von 50 % oder mehr, 60 % oder mehr oder 80 % oder mehr, besonders bevorzugt von 95 % oder mehr, umfasst bzw. daraus besteht
und/oder
die pflanzliche Chalconisomerase eine Aminosäuresequenz gemäß SEQ ID NO:**8** (Aminosäuresequenz der Chalconeisomerase aus *Medicago sativa)* oder SEQ ID NO:**9** (Aminosäuresequenz der Chalcone-flavonone isomerase 1 aus *Arabidopsis thaliana)* oder eine Aminosäuresequenz mit einer Aminosäuresequenz-Identität von 40 % oder mehr zu SEQ ID NO:**8** oder SEQ ID NO:**9**, vorzugsweise von 50 % oder mehr, 60 % oder mehr oder 80 % oder mehr, besonders bevorzugt von 95 % oder mehr, umfasst bzw. daraus besteht
und/oder
die bakterielle Enoat-Redukatase eine Aminosäuresequenz gemäß SEQ ID NO:**4** (Aminosäuresequenz der bakteriellen ERED aus *E. ramulus* DSM 16296) oder eine Aminosäuresequenz mit einer Aminosäuresequenz-Identität von 40 % oder mehr zu SEQ ID NO:**4**, vorzugsweise von 50 % oder mehr, 60 % oder mehr oder 80 % oder mehr, besonders bevorzugt von 95 % oder mehr, umfasst bzw. daraus besteht.

Im Rahmen der vorliegenden Erfindung ist die "Aminosäuresequenz-Identität" bevorzugt anhand des Waterman-Smith-Algorhytmus mit einer Lückeneröffnungs-Strafe ("gap open penalty") von 10, einer Lückenvergrößerungsstrafe ("gap extension penalty") von 0,5 und der BLOSUM62-Matrix zu bestimmen (bezüglich des Waterman-Smith-Algorhytmus siehe beispielsweise Smith, T.F. and Waterman, M.S., Identification of common molecular subsequences, Journal of Molecular biology (1981), 147:195-197; online implementiert über die entsprechende Werkzeugseite des EMBL).

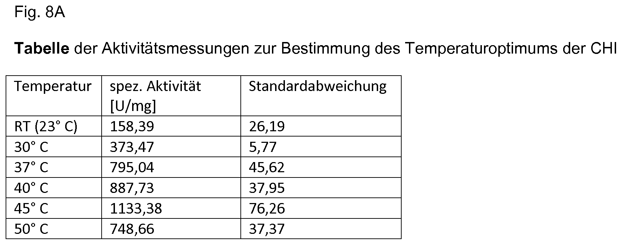

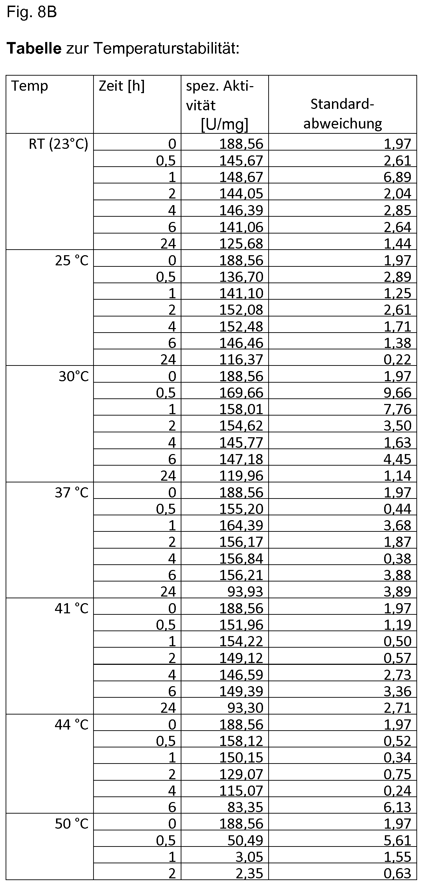

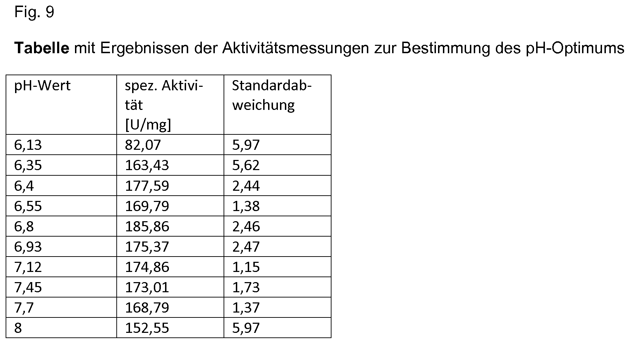

Für die Zwecke der vorliegenden Erfindung besonders bevorzugt ist die Verwendung einer Chalconisomerase, die eine, mehrere oder sämtliche der folgenden Eigenschaften und/oder eine Temperatur- und pH-Stabilität gemäß Fig. 8A und Fig. 9 aufweist:

| **K_{M} [µmol/l]** | **Vₘₐₓ [U/mg]** | **k_{cat} [s⁻¹]** | **k_{cat} / K_{M} [l*mol⁻¹*s⁻¹]** |
|---|---|---|---|
| 36,83 | 107,3 | 416,7 | 1,13 * 107 |

Für die Zwecke der vorliegenden Erfindung bevorzugt ist die Verwendung einer Enoat-Reduktase, die eine, mehrere oder sämtliche der folgenden Eigenschaften aufweist:
- Proteingröße von 74,455 kDa
- wird sowohl in der löslichen als auch in der unlöslichen Proteinfraktion nach bis zu 20h unter anoxischen Bedingungen bei verschiedenen Temperaturen exprimiert.

Im Folgenden werden weitere, erfindungsgemäß besonders bevorzugte Einzelheiten eines erfindungsgemäßen Verfahrens zur Herstellung von Phloretin beschrieben.

Betreffend das Bereitstellen eines transgenen Mikroorganismus (wie hierin beschrieben) ist anzumerken, dass grundsätzlich jede dem Fachmann geläufige Methode verwendet werden kann, um die hierin beschriebenen Nukleinsäure-Abschnitte (a), (a') und (b) bzw. die hierin beschriebenen Transgene in die Mikroorganismen einzuschleusen, z.B. grundsätzlich durch Konjugation, Transduktion oder Transformation, vorzugsweise durch Hitzeschock-Behandlung, Elektroporation, Konjugation, Gene-gun, Litium-Acetat-Methode oder Transduktion. Generell ist es im Rahmen der vorliegenden Erfindung jedoch bevorzugt, wenn die Nukleinsäure-Abschnitte (a), (a') und (b) bzw. die beschriebenen Transgene mittels eines Vektors, insbesondere eines Plasmidvektors, vorzugsweise eines erfindungsgemäßen Vektors wie hierin beschrieben (s. unten), eingeschleust werden. Methoden hierzu sind dem Fachmann hinreichend bekannt.

Ein transgener Mikroorganismus im Sinne der vorliegenden Erfindung kann eine oder mehrere Kopien der eingeschleusten Nukleinsäure-Abschnitte bzw. der hierin beschriebenen Transgene enthalten.

Methoden, um auf Basis der eingeschleusten Nukleinsäure-Abschnitte bzw. der Transgene eine Expression der gewünschten Aminosäuresequenzen bzw. der gewünschten Enzyme zu ermöglichen, sind dem Fachmann ebenfalls hinreichend bekannt, z.B. unter Verwendung eines regulatorischen Elements, insbesondere eines Promotors (s. hierzu auch die beigefügten Beispiele).

Wie oben beschrieben wird bzw. werden in Schritt (ii) eines erfindungsgemäßen Verfahrens ein oder mehrere Flavanone und/oder eine oder mehrere Vorstufe(n) oder Derivat(e) davon zu dem transgenen Mikroorganismus hinzugefügt, wobei der transgene Mikroorganismus unter Bedingungen kultiviert wird, die die Umsetzung des bzw. desr Flavanone und/oder der Vorstufe(n) oder des Derivats bzw. der Derivate davon, insbesondere von Naringin und/oder der Vorstufe bzw. des Derivats von Naringin, zu einem Dihydrochalkon, insbesondere zu Phloretin, ermöglichen.

Wie oben beschrieben wird in Schritt (ii) eines erfindungsgemäßen Verfahrens besonders bevorzugt Naringin und/oder eine Vorstufe oder ein Derivat von Naringin zu dem transgenen Mikroorganismus hinzugefügt, wobei der transgene Mikroorganismus unter Bedingungen kultiviert wird, die die Umsetzung des Naringin und/oder der Vorstufe bzw. des Derivats von Naringin zu Phloretin ermöglichen.

Gemäß einer bevorzugten Durchführung eines erfindungsgemäßen Verfahrens (wie hierin beschrieben) werden die (transgenen) Mikroorgansimen zunächst, d.h. vor Schritt (ii), unter aeroben Bedingungen, vorzugsweise bis zum Erreichen einer maximalen Biomassenkonzentration, kultiviert. Dabei sollte die OD₆₀₀ bevorzugt wenigstens im Bereich von 8 bis 15 oder darüber liegen, vorzugsweise im Bereich von 5 bis 190, insbesondere im Bereich von 10 bis 180, bevorzugt im Bereich von 15 bis 170. Anschließend werden die Mikroorganismen in Schritt (ii) bevorzugt unter anaeroben Bedingungen kultiviert, wobei die Expression der gewünschten Aminosäuresequenzen bzw. der gewünschten Enzyme auf Basis der eingeschleusten Nukleinsäure-Abschnitte bzw. der eingeschleusten Transgene erfolgt, beispielsweise angeregt mittels Induktion durch IPTG und/oder Lactose (bei Verwendung eines entsprechenden, geeigneten Promotors bzw. eines entsprechenden, geeigneten Expressionssystems).

Grundsätzlich ist es erfindungsgemäß bevorzugt, wenn die Inkubation in Schritt (ii) zumindest teilweise oder vollständig unter anaeroben Bedingungen erfolgt.

Je nach Mikroorganismus kann der Fachmann in Schritt (ii) für die Zwecke der vorliegenden Erfindung geeignete Umgebungsbedingungen schaffen und insbesondere ein geeignetes (Kultivierungs-)Medium bereitstellen. Die Kultivierung erfolgt vorzugsweise in LB-oder TB-Medium. Alternativ kann ein (komplexeres) Medium bestehend oder umfassend pflanzliche Rohstoffe, insbesondere aus Zitrus-, Grapefruit- und Orange-Pflanzen, verwendet werden. Die Kultivierung erfolgt beispielsweise bei einer Temperatur von mehr als 20 °C, bevorzugt von mehr als 25 °C, insbesondere von mehr als 30 °C (bevorzugt im Bereich von 30 bis 40 °C), was insbesondere die Phloretinbildung begünstigen bzw. die Ausbeute erhöhen kann. Weiterhin kann auch eine Temperatur zur Induktion (vgl. oben) von weniger als 40 °C, insbesondere von weniger als 35 °C (bevorzugt im Bereich von 20 bis 30 °C), die Phloretinbildung begünstigen bzw. die Ausbeute erhöhen.

Naringin bzw. die Vorstufe oder das Derivat davon wird bezogen auf das (Kultivierungs-) Medium, das die transgenen Mikrorganismen enthält, in Schritt (ii) vorzugsweise in einer Menge von 0,1 mM bis 100 mM (mMol / L), bevorzugt von 0,5 bis 95 mM, besonders bevorzugt von 1 bis 90 mM, zu dem transgenen Mikroorganismus hinzugefügt. Hierbei können geeignete (Ko-)Solventien zum Einsatz kommen.

Falls zur Induktion (z.B. des lac-operons) ein oder mehrere geeignete Induktoren, z.B. IPTG oder Lactose , verwendet werden (vgl. oben), ist es bevorzugt, den Induktor bezogen auf das (Kultivierungs-)Medium, das die transgenen Mikrorganismen enthält, in Schritt (ii) in einer Menge von 0,001 bis 1 mM, bevorzugt von 0,005 bis 0,9 mM, besonders bevorzugt von 0,01 bis 0,8 mM, einzusetzen, da hierbei besonders gute Ausbeuten erzielt werden können.

Betreffend das optionale Isolieren sowie gegebenenfalls Aufreinigen von Phloretin: Hierbei können beispielsweise Extraktionen mit organischen Lösemitteln (bevorzugt ausgewählt aus der folgenden Liste: Isobutan, 2-Propanol, Toluol, Methylacetat, Cyclohexan, 2-Butanol, Hexan, 1-Propanol, Light petroleum, 1,1,1,2-Tetrafluorethan, Methanol, Propan, 1-Butanol, Butan, Ethylmethylketon, Ethylacetat, Diethylether, Ethanol, Dibutylether, CO2, tert. Butylmethylether, Aceton, Dichloromethan und N2O), besonders bevorzugt solchen, die mit Wasser eine optisch erkennbare Phasengrenze ausbilden, vorgenommen werden. Hiernach kann sich eine Entfernung des Restwassers im Lösemittel sowie die Entfernung des Lösemittels selbst anbieten, an die sich wiederum ein Rücklösen des (z.B.) Phloretins in einem (ggf. anderen) Lösemittel anschließen kann, welches z.B. für eine gegebenenfalls nachfolgende Kristallisation und Trocknung des Produkts geeignet ist. Alternativ oder ergänzend kann eine adsorptive, eine destillative und/oder eine chromatographische Aufreinigung erfolgen.

Weitere Einzelheiten eines erfindungsgemäßen Verfahrens ergeben sich aus den beigefügten Beispielen.

Ein weiterer Aspekt der vorliegenden Erfindung betrifft einen transgenen Mikroorganismus, enthaltend einen Nukleinsäure-Abschnitt (a), umfassend oder bestehend aus einem für eine bakterielle Chalconisomerase kodierenden Gen (als Transgen), und/oder einen Nukleinsäure-Abschnitt (a'), umfassend oder bestehend aus einem für eine pflanzliche Chalconisomerase kodierenden Gen (als Transgen), sowie einen Nukleinsäure-Abschnitt (b), umfassend oder bestehend aus einem für eine bakterielle Enoat-Reduktase kodierenden Gen (als weiteres Transgen), wobei der transgene Mikroorganismus kein Mikroorganismus der Ordnung Clostridiales ist und wobei der Mikroorganismus Chalconisomerase- und Enoat-Reduktase-Aktivität aufweist, jedoch keine Phloretinhydrolase-Aktivität.

Dabei gilt bezüglich der verwendeten Begriffe das oben für diese Begriffe Gesagte entsprechend. Bevorzugte erfindungsgemäße Mikroorganismen ergeben sich entsprechend aus den obigen Ausführungen zu im Rahmen eines erfindungsgemäßen Verfahrens bevorzugt einzusetzenden Mikroorganismen.

Der erfindungsgemäße Mikroorganismus weist wenigstens eine Chalconisomerase- und eine Enoat-Reduktase-Aktivität, jedoch keine Phloretinhydrolase-Aktivität auf. Entsprechendes gilt für im Rahmen eines erfindungsgemäßen Verfahrens einzusetzende Mikroorganismen (wie oben beschrieben).

Der Mikroorganismus ist kein *Eubacterium ramulus,* und kein Mikroorganismus der Ordnung Clostridiales, weiter bevorzugt kein Mikroorganismus der Klasse Clostridia, besonders bevorzugt kein Mikroorganismus des Phylus der Firmicutes, und ist besonders bevorzugt ausgewählt aus der Gruppe bestehend fakultativ anaeroben Mikroorganismen, insbesondere fakultativ aeroben Bakterien, bevorzugt Proteobakterien, insbesondere Enterobakterien, zum Beispiel der Gattung *Escherichia,* bevorzugt *E. coli,* insbesondere *E. coli* Rosetta, *E. coli* BL21 und *E. coli* SE1, und Hefen, zum Beispiel S. *cerevesiae* und *P. pastoris.* Im Übrigen gilt das oben für im Rahmen eines erfindungsgemäßen Verfahrens bevorzugt einzusetzende Mikroorganismen Gesagte entsprechend.

Dementsprechend ist ein erfindungsgemäßer Mikroorganismus besonders bevorzugt, wobei
das für eine bakterielle Chalconisomerase kodierende Gen für eine Chalconisomerase aus einem Mikroorganismus aus dem Phylus der Firmicutes, vorzugsweise der Klasse Clostridia, insbesondere der Ordnung Clostridiales, besonders bevorzugt für eine Chalconisomerase aus *E. ramulus,* kodiert,
und/oder
das für eine pflanzliche Chalconisomerase kodierende Gen für eine Chalconisomerase aus *A. thaliana* oder M. *sativa* kodiert (für weitere bevorzugte Quellen vgl. oben),
und/oder
das für eine bakterielle Enoat-Reduktase kodierende Gen für eine Enoat-Reduktase aus einem Mikroorganismus aus dem Phylus der Firmicutes, vorzugsweise der Klasse Clostridia, insbesondere der Ordnung Clostridiales, besonders bevorzugt für eine Enoat-Reduktase aus *E. ramulus* kodiert.

Ferner ist es bevorzugt, wenn
der Nukleinsäure-Abschnitt (a) eine Nukleotidsequenz gemäß SEQ ID NO:**1** oder eine Nukleotidsequenz mit einer Nukleotidsequenz-Identität von 40 % oder mehr zu SEQ ID NO:**1**, vorzugsweise von 50 % oder mehr, 60 % oder mehr oder 80 % oder mehr, besonders bevorzugt von 95 % oder mehr, umfasst oder daraus besteht
und/oder
der Nukleinsäure-Abschnitt (a') eine Nukleotidsequenz gemäß SEQ ID NO:**6** oder SEQ ID NO:**7** oder eine Nukleotidsequenz mit einer Nukleotidsequenz-Identität von 40 % oder mehr zu SEQ ID NO:**6** oder SEQ ID NO:**7**, vorzugsweise von 50 % oder mehr, 60 % oder mehr oder 80 % oder mehr, besonders bevorzugt von 95 % oder mehr, umfasst oder daraus besteht
und/oder
der Nukleinsäure-Abschnitt (b) eine Nukleotidsequenz gemäß SEQ ID NO**:2** oder SEQ ID NO**:5** oder eine Nukleotidsequenz mit einer Nukleotidsequenz-Identität von 40 % oder mehr zu SEQ ID NO**:2** oder SEQ ID NO**:5**, vorzugsweise von 50 % oder mehr, 60 % oder mehr oder 80 % oder mehr, besonders bevorzugt von 95 % oder mehr, umfasst oder daraus besteht.

Außerdem ist es bevorzugt, wenn
die bakterielle Chalconisomerase eine Aminosäuresequenz gemäß SEQ ID NO:**3** oder eine Aminosäuresequenz mit einer Aminosäuresequenz-Identität von 40 % oder mehr zu SEQ ID NO:**3**, vorzugsweise von 50 % oder mehr, 60 % oder mehr oder 80 % oder mehr, besonders bevorzugt von 95 % oder mehr, umfasst bzw. daraus besteht
und/oder
die pflanzliche Chalconisomerase eine Aminosäuresequenz gemäß SEQ ID NO:**8** oder SEQ ID NO:**9** oder eine Aminosäuresequenz mit einer Aminosäuresequenz-Identität von 40 % oder mehr zu SEQ ID NO:**8** oder SEQ ID NO**:9**, vorzugsweise von 50 % oder mehr, 60 % oder mehr oder 80 % oder mehr, besonders bevorzugt von 95 % oder mehr, umfasst bzw. daraus besteht
und/oder
die bakterielle Enoat-Redukatase eine Aminosäuresequenz gemäß SEQ ID NO**:4** oder eine Aminosäuresequenz mit einer Aminosäuresequenz-Identität von 40 % oder mehr zu SEQ ID NO**:4**, vorzugsweise von 50 % oder mehr, 60 % oder mehr oder 80 % oder mehr, besonders bevorzugt von 95 % oder mehr, umfasst bzw. daraus besteht.

Für die Bestimmung der Nukleotidsequenz- und Aminosäuresequenz-Identität gilt dabei das oben Gesagte entsprechend.

Hierin beschrieben wird auch ein Vektor, d.h. ein Transportvesikel ("Genfähre") zur Übertragung von Fremd-Nukleinsäure(n) in eine Empfängerzelle, insbesondere ein Plasmidvektor, der das Klonieren eines oder mehrerer Nukleinsäure-Abschnitte ermöglicht, enthaltend einen Nukleinsäure-Abschnitt (a), umfassend oder bestehend aus einem für eine bakterielle Chalconisomerase kodierenden Gen, und/oder einen Nukleinsäure-Abschnitt (a'), umfassend oder bestehend aus einem für eine pflanzliche Chalconisomerase kodierenden Gen, und/oder einen Nukleinsäure-Abschnitt (b), umfassend oder bestehend aus einem für eine bakterielle Enoat-Reduktase kodierenden Gen. Dabei ist es bevorzugt, wenn der Vektor sowohl einen Nukleinsäure-Abschnitt (a) und/oder (a'), als auch einen Nukleinsäure-Abschnitt (b) enthält.

Neben Nukleinsäure-Abschnitt(en) (a), (a') und/oder (b) enthält ein solcher Vektor für die Zwecke der vorliegenden Erfindung gegebenenfalls weitere übliche Bestandteile, insbesondere solche, die die Expression der hierin beschriebenen Transgene in Mikroorganismen, insbesondere in solchen wie oben beschrieben, verbessern oder gegebenenfalls erst ermöglichen. Grundsätzlich enthält ein solcher Vektor vorzugsweise zudem einen oder mehrere weitere Bestandteile bzw. Elemente ausgewählt aus der Gruppe bestehend aus Promotor, ori-Sequenz, Sequenz zur affinitätschromatographischen Reinigung, Selektionsmarker, Operatorsequenz, Terminator, ribosomale Bindestellen, Proteasespaltsequenz, Rekombinationsbindestellen, Sequenzen von Fusionsproteinen und Chaperon-sequenzen.

Auch bei den Vektoren wie oben beschrieben ist es bevorzugt, wenn
das für eine bakterielle Chalconisomerase kodierende Gen für eine Chalconisomerase aus einem Mikroorganismus aus dem Phylus der Firmicutes, vorzugsweise der Klasse Clostridia, insbesondere der Ordnung Clostridiales, besonders bevorzugt für eine Chalconisomerase aus *E. ramulus,* kodiert
und/oder
das für eine pflanzliche Chalconisomerase kodierende Gen für eine Chalconisomerase aus *A. thaliana* oder M. *sativa* kodiert (für weitere bevorzugte Quellen vgl. oben),
und/oder
das für eine bakterielle Enoat-Reduktase kodierende Gen für eine Enoat-Reduktase aus einem Mikroorganismus aus dem Phylus der Firmicutes, vorzugsweise der Klasse Clostridia, insbesondere der Ordnung Clostridiales, besonders bevorzugt für eine Enoat-Reduktase aus *E. ramulus* kodiert.

Besonders bevorzugt ist es, wenn
der Nukleinsäure-Abschnitt (a) eine Nukleotidsequenz gemäß SEQ ID NO:**1** oder eine Nukleotidsequenz mit einer Nukleotidsequenz-Identität von 40 % oder mehr zu SEQ ID NO:**1**, vorzugsweise von 50 % oder mehr, 60 % oder mehr oder 80 % oder mehr, besonders bevorzugt von 95 % oder mehr, umfasst oder daraus besteht
und/oder
der Nukleinsäure-Abschnitt (a') eine Nukleotidsequenz gemäß SEQ ID NO:**6** oder SEQ ID NO:**7** oder eine Nukleotidsequenz mit einer Nukleotidsequenz-Identität von 40 % oder mehr zu SEQ ID NO:**6** oder SEQ ID NO:**7**, vorzugsweise von 50 % oder mehr, 60 % oder mehr oder 80 % oder mehr, besonders bevorzugt von 95 % oder mehr, umfasst oder daraus besteht
und/oder
der Nukleinsäure-Abschnitt (b) eine Nukleotidsequenz gemäß SEQ ID NO:**2** oder SEQ ID NO:**5** oder eine Nukleotidsequenz mit einer Nukleotidsequenz-Identität von 40 % oder mehr zu SEQ ID NO:**2** oder SEQ ID NO:**5**, vorzugsweise von 50 % oder mehr, 60 % oder mehr oder 80 % oder mehr, besonders bevorzugt von 95 % oder mehr, umfasst oder daraus besteht.

Weiterhin ist es bevorzugt, wenn
die bakterielle Chalconisomerase eine Aminosäuresequenz gemäß SEQ ID NO:**3** oder eine Aminosäuresequenz mit einer Aminosäuresequenz-Identität von 40 % oder mehr zu SEQ ID NO:**3**, vorzugsweise von 50 % oder mehr, 60 % oder mehr oder 80 % oder mehr, besonders bevorzugt von 95 % oder mehr, umfasst bzw. daraus besteht
und/oder
die pflanzliche Chalconisomerase eine Aminosäuresequenz gemäß SEQ ID NO:**8** oder SEQ ID NO:**9** oder eine Aminosäuresequenz mit einer Aminosäuresequenz-Identität von 40 % oder mehr zu SEQ ID NO:**8** oder SEQ ID NO:**9**, vorzugsweise von 50 % oder mehr, 60 % oder mehr oder 80 % oder mehr, besonders bevorzugt von 95 % oder mehr, umfasst bzw. daraus besteht
und/oder
die bakterielle Enoat-Redukatase eine Aminosäuresequenz gemäß SEQ ID NO:**4** oder eine Aminosäuresequenz mit einer Aminosäuresequenz-Identität von 40 % oder mehr zu SEQ ID NO:**4**, vorzugsweise von 50 % oder mehr, 60 % oder mehr oder 80 % oder mehr, besonders bevorzugt von 95 % oder mehr, umfasst bzw. daraus besteht.

Auch hierbei gilt für die Bestimmung der Nukleotidsequenz- und Aminosäuresequenz-Identität das oben Gesagte entsprechend.

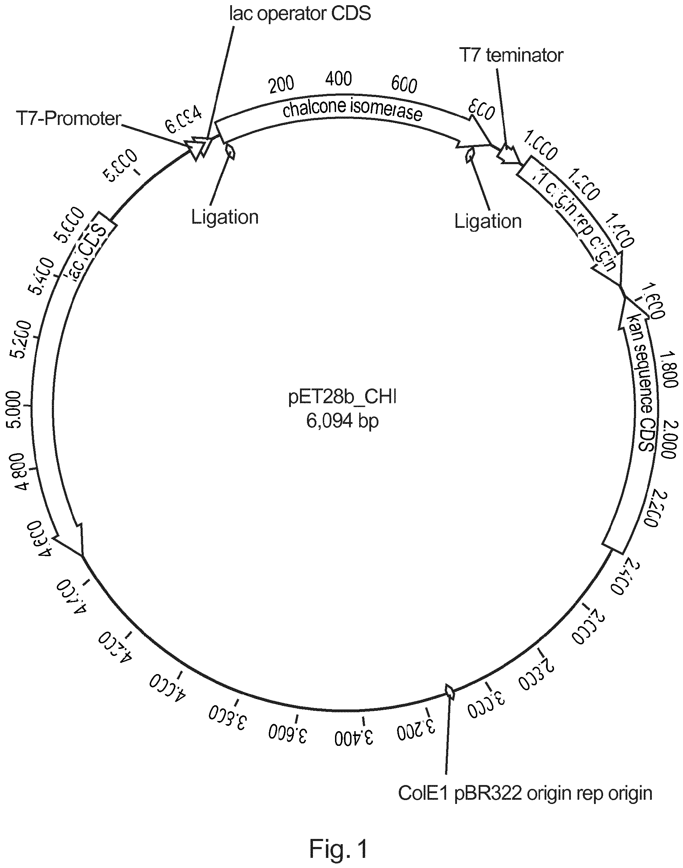

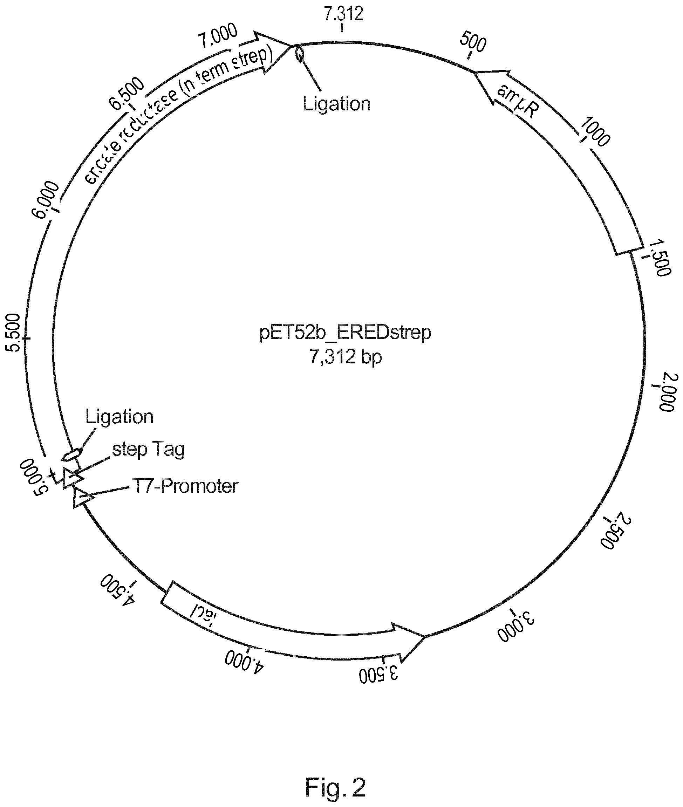

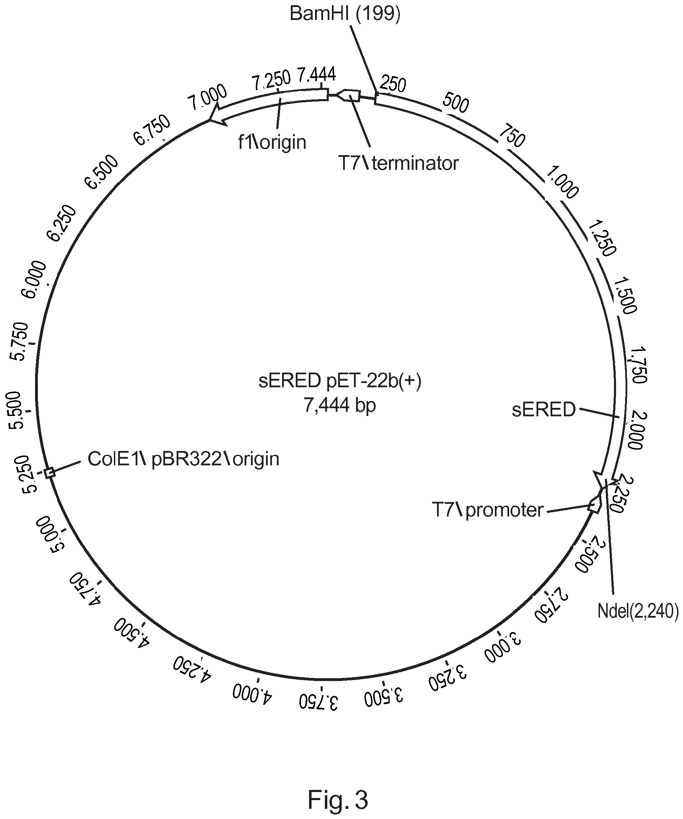

Besonders bevorzugte und für die Zwecke der Erfindung besonders geeignete Vektoren und Bestandteile bzw. Elemente davon ergeben sich auch aus den beigefügten Beispielen und Figuren (Fig. 1: Plasmid pET52b_EREDstrep zur heterologen Expression und Charakterisierung der ERED aus *E. ramulus* DSM 16296; Fig. 2: Plasmid pET28b_CHI zur heterologen Expression und Charakterisierung der CHI aus *E. ramulus* DSM 16296; Fig 3: Plasmid pET22b_ERED zur heterologen Expression und Charakterisierung der ERED aus *E. ramulus* DSM 16296).

Die vorliegende Erfindung betrifft auch einen transgenen Mikroorganismus (wie oben beschrieben) in Form einer Wirtszelle, enthaltend ein oder mehrere identische oder unterschiedliche Vektoren wie hierin beschrieben. Erfindungsgemäß bevorzugt ist eine Wirtszelle, die sowohl ein oder mehrere Vektoren mit einem Nukleinsäure-Abschnitt (a), umfassend oder bestehend aus einem für eine bakterielle Chalconisomerase kodierenden Gen, und/oder einem Nukleinsäure-Abschnitt (a'), umfassend oder bestehend aus einem für eine pflanzliche Chalconisomerase kodierenden Gen, als auch ein oder mehrere Vektoren mit einem Nukleinsäure-Abschnitt (b), umfassend oder bestehend aus einem für eine bakterielle Enoat-Reduktase kodierenden Gen, enthält. Besonders bevorzugt ist eine Wirtszelle, die ein oder mehrere Vektoren mit sowohl einem Nukleinsäure-Abschnitt (a) und/oder (a'), als auch einem Nukleinsäure-Abschnitt (b) enthält.

Bei einer erfindungsgemäßen Wirtszelle handelt es sich um einen erfindungsgemäß einzusetzenden bzw. einen erfindungsgemäßen Mikroorganismus (wie oben beschrieben). Die hierin beschriebenen erfindungsgemäßen Wirtszellen bzw. erfindungsgemäßen oder erfindungsgemäß einzusetzenden Mikroorganismen sind bzw. dienen vorzugsweise als (Produktions-)Stamm zur biotechnologischen Herstellung hierin beschriebener Dihydrochalkone, insbesondere von Phloretin (wie oben beschrieben).

Nachfolgend wird die vorliegende Erfindung anhand von Beispielen näher erläutert, wobei diese den Gegenstand der beigefügten Patentansprüche jedoch nicht beschränken.

### Beispiel 1: Bereitstellen transgener Mikroorganismen (vgl. Schritt (i))

### 1.1 CHI:

Anhand der identifizierten Gensequenz der Chalconisomerase aus *E. ramulus* wurden zwei Primer erstellt, die zur Vervielfältigung der genomischen DNA mittels PCR verwendet wurden. Hierbei wurden mit Hilfe der Primer vor dem Gen eine Restriktionsschnittstellen für Kpn1 und BamH1 sowie hinter dem Gen eine Schnittstelle für Not1 an die Zielsequenz angefügt, die für die Ligation des Genabschnitts in den Zielvektor verwendet wurden.

Verwendete Primer (Sequenzabschnitte, die direkt an das Gen binden sind kursiv dargestellt):
forward: CTAATCGGATCCGGTACC*ATGGCAGATTTCAAATTCGAACCAATG*
reverse: TCAGTAGCGGCCG*CTTATCTCATGGTGATGTATCCACGATAATT*

Das Einfügen des entstandenen DNA-Fragmentes des Chalconisomerase-Gens wurde mittels TOPO TA Cloning® (von Invitrogen, Carlsbad, Kalifornien, USA) in den Vektor pCR®2.1-TOPO® eingefügt. Nach erfolgreicher Transformation dieses Konstruktes wurde das Chalconisomerase-Gen aus diesem Vektor über Nco1 und Not1 ausgeschnitten und in den ebenfalls mit Nco1 und Not1 geschnittenen Zielvektor pET28b eingefügt. Das Plasmid wurde in *E. coli* Rosetta eingeschleust und dort erfolgreich exprimiert.

Nach erfolgreicher Transformation wurde die Sequenzidentität über eine Sequenzierung bestätigt.

### 1.2 ERED:

Die Enoatreduktase wurde mit folgenden spezifischen Primern aus der genomischen DNA amplifiziert (Sequenzabschnitte, die direkt an das Gen binden, sind kursiv dargestellt):
forward: GATCCTCGAG*ATGGCAGAAAAAAATCAGTATTTTCCACA*
reverse: *GATCAAGCTTAGATAATTTCCATTGCTGCGGTCCA*

Dabei wurde vor dem Gen eine Schnittstelle für SacI und hinter dem Gen eine Schnittstelle für HindIII eingefügt, um eine spätere Klonierung zu ermöglichen.

Auch dieses Fragment wurde mit dem TOPO TA Cloning®-Kit weiterbearbeitet. Die Sequenzidentität des resultierenden Klons (mit Vektor pCR®2.1-TOPO® und darin enthaltenem Gen für die Enoatreduktase) wurde über eine Sequenzierung bestätigt.

Im Anschluss wurde das Gen aus diesem Plasmid heraus mit Primern amplifziert, die vor dem Gen eine KpnI Schnittstelle einfügten (Sequenzabschnitte, die direkt an das Gen binden, sind kursiv dargestellt; die in den forward-Primer (der keine Sequenzabschnitte beinhaltet, die direkt an das Gen binden) eingefügte KpnI-Schnittstelle wurde fett markiert):
forward: AGTGTGATGGGTACCTGCAGAATTCGCC
reverse: *GATCAAGCTTAGATAATTTCCATTGCTGCGGTCCA* (vgl. oben)

Im Vektor pCR®2.1-TOPO® ist ebenfalls eine Sacl-Schnittstelle enthalten, die für die weitere Klonierung verwendet wurde.

Nach der PCR erfolgte ein Verdau dieses PCR-Produktes mit Sac1 und Kpn1 und anschließend die Ligation in das ebenfalls mit Sac1 und Kpn1 verdaute Plasmid pET52b. Das Genkonstrukt wurde in *E. coli* Rosetta exprimiert.

Über eine Sequenzierung wurde bestätigt, dass das Gen der Enoatreduktase erfolgreich in das Plasmid pET52b ligiert wurde, wobei der eingefügte N-terminale Strep-tag vorhanden blieb, der eine hochspezifische Proteinaufreinigung ermöglicht.

Im Rahmen eines weiteren Ansatzes wurde eine codonoptimierte Sequenz mit den Schnittstellen Nde1 und BamH1 in den Vektor pET-22b ligiert und in *E. coli* BL21 exprimiert.

### 1.3 antibiotikafreie Expression:

Weiterhin können die Genabschnitte der CHI über die Schnittstellen BamHI und XHO1 in das Plasmid pET22b mit dem synthetischen ERED-Gen integriert werden.

Dafür wird das CHI-Gen über eine PCR mit dem forward-Primer GTCTAGGATCCAGAAATAATTTTGTTTAACTTTAAGAAGGAGA und dem pET-rP-Primer CTAGTTATTGCTCAGCGG amplifiziert, wobei die Xba1-Schnittstelle vor dem CHI-Gen über den forward-Primer in eine BamHI-Schnittstelle mutiert wird. Danach wird das Konstrukt aus beiden Genen über die Schnittstellen Xba1 und Xho1 aus dem Plasmid geschnitten und in das mit Xba1 und Xho1 vorbereitete Plasmid pStaby1.2 ligiert.

Anschließend kann eine antibiotikafreie Expression durch das StabyExpress-System erfolgen.

### Beispiel 2: Biotechnologische Herstellung von Phloretin (vgl. Schritt (ii))

TB-Medium bestehend aus 24 g Hefeextrakt, 12 g Trypton und 4 g Glycerin wird auf 900 ml aufgefüllt und bei 121 °C für 15 Minuten autoklaviert. Eine separat hergestellte Salzlösung (0,72 M an K₂HPO₄ und 0,17 M an KH₂PO₄) wird bei den gleichen Bedingungen autoklaviert. Im Anschluss daran werden 100 ml der Salzlösung zu 900 ml des sterilen TB-Mediums zugegeben. LB-Medium bestehend aus 5 g Hefeextrakt, 10 g Trypton und 10 g NaCl wird auf 1000 ml mit destilliertem Wasser aufgefüllt und für 15 Minuten bei 121 °C autoklaviert.

Die z.B. gemäß Beispiel 1 transformierten *E. coli* Rosetta bzw. *E. coli* BL21 (vgl. oben) werden zunächst in 250 ml Erlenmeyerkolben (mit Schikane), die mit 50 ml LB-Medium gefüllt sind, für etwa 8 Stunden bei 37 °C und 180 rpm kultiviert. Von dieser Kultur werden dann 7 ml entnommen und ein Minifermenter inokuliert, in dem sich 700 ml TB-Medium (wie oben beschrieben) befinden. Die Kultur wird über Nacht bei 25 °C mit 150 bis 200 rpm und 40% Sauerstoffsättigung zum Erreichen einer maximalen Biomassekonzentration angezogen.

Vor der (Über-)Expression der eingeschleusten Nukleinsäure-Abschnitte bzw. der eingeschleusten Transgene wird die Luftzufuhr beendet und der vorhandene Sauerstoff wird mit Stickstoff ausgetrieben.

Zur Induktion der (Über-)Expression wird zunächst eine IPTG-Konzentration von 1 mM im Medium eingestellt und dann 7 ml einer Naringenin-Lösung (100mM in DMSO) zugegeben.

Im Anschluss wird für mindestens 8 - 20 Stunden unter anoxischen Bedingungen während der Expressionsphase weiter kultiviert.

Beispiel 3: Charakterisierung der exprimierten CHI

Nach der Expression von z.B. pET28b_CHI in *E. coli* (vgl. oben) kann CHI sowohl in den löslichen als auch in den unlöslichen Fraktionen nachgewiesen werden.

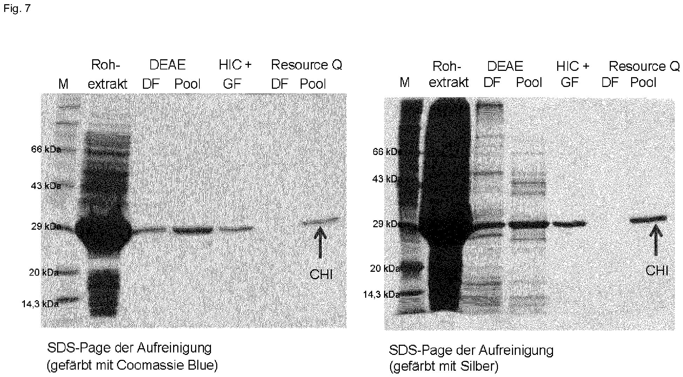

Nach einer Aufreinigung mit z.B. Anionenaustauschchromatographie, Hydrophober Interaktionschromatographie, Größenausschlusschromatographie und/oder Resource Q-Aufreinigung kann die CHI zur Charakterisierung isoliert werden (in Fig. 7 sind beispielhaft die Ergebnisse der einzelnen Aufreinigungsschritte nach Expression des Vektors pET28b_CHI in *E.coli* gezeigt).

Im Rahmen eigener Untersuchungen ergaben sich die in Tabelle 1 genannten Werte:

**Tabelle 1: Charakterisierung der Enzymaktivität der CHI nach Expression des Vektors pET28b_CHI in E. coli**

| KM [µmol/l] | Vmax [U/mg] | kcat [s-1] | kcat / KM [l*mol-1*s-1] |
|---|---|---|---|
| 36,83 | 107,3 | 416+,7 | 1,13 * 107 |

### Beispiel 4: (Weitere) Charakterisierung der exprimierten Enzyme CHI und ERED

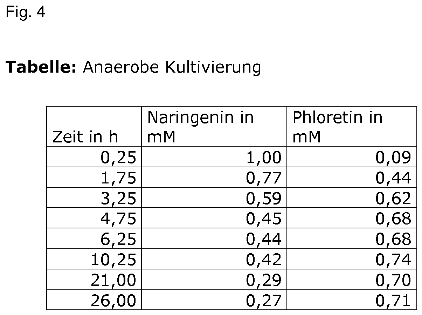

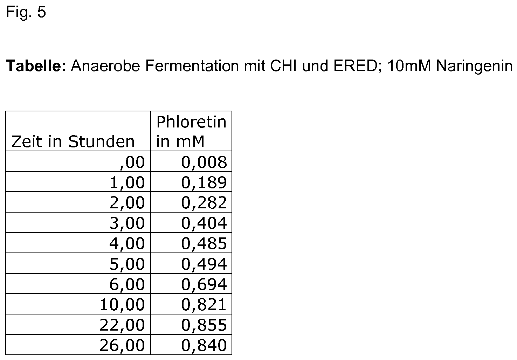

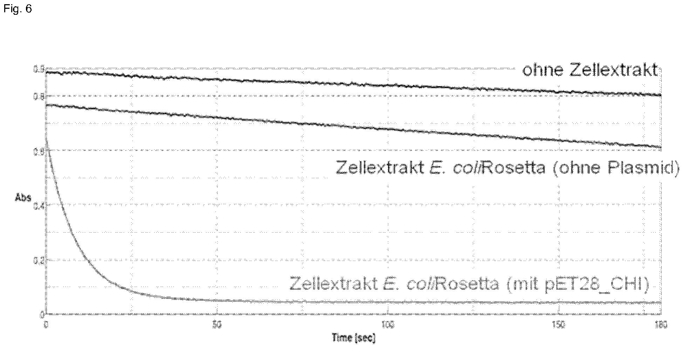

Die Tabellen in Fig. 4 und 5 zeigen (weitere) Ergebnisse der Charakterisierung der exprimierten Enzyme CHI und ERED (aus *E. ramulus* DSM 16296 bei anaerober Umsetzung (vgl. oben)).

Die Enyzme CHI und ERED (aus *E. ramulus* DSM 16296) wurden in einem für *E. coli* geeignetem Vektorsystem separat zur Expression gebracht (vgl. oben). Anschließend kann deren Aktivität - nach Zugabe von Naringenin in ein definiertes Kulturmedium - mittels HPLC bestimmt werden.

Die Werte der Tabelle in Fig. 4 zeigen im zeitlichen Verlauf die Bildung von Phloretin (unter Abnahme der Naringenin-Konzentration), was auf die Aktivität der Enzyme CHI und ERED hinweist.

Die Werte der Tabelle in Fig. 5 zeigen (weitere) Ergebnisse bezüglich der Chalkonisomerase-Aktivität der exprimierten CHI, erhalten durch photometrische Bestimmung (bei 368 nm) im Zusammenhang mit dem Abbau von Naringeninchalcon (zu Phloretin).

Im Rahmen einer photometrischen Bestimmung konnte - zur weiteren Untersuchung der CHI-Aktivität - im zeitlichen Verlauf die Abnahme der Konzentration von Naringeninchalcon in einem zellfreien, gepufferten Proteinrohextrakt beobachtet werden. Ein Kontrollversuch (Plasmid ohne CHI) ergab keine Abnahme des als Markersubstanz verwendeten Naringeninchalcons.

### Beispiel 5: Ergebnisse aus (Vergleichs-)Expressionsversuchen mit E. ramulus

*E. ramulus* (DSMZ 16296) wurde nach Herles et al. (Arch Microbiol (2004) 181 : 428-434) anaerob in ST Medium kultiviert. Zu dessen Herstellung wurden 9 g tryptisch behandeltes Fleischpepton, 1 g Pepton, 3 g Fleischextraktt, 4 g Hefeextrakt, 6 g Glucose, 3 g Natriumchlorid, 2 g Di-Natriumhydrogenphosphat, 0,5 ml Tween 80, 0,25 g Cystine, 0,25 g Cystein-HCl, 0,1 g Magnesiumsulfat-heptahydrat, 5 mg Eisensulfat-heptahydrat und 3.4 mg Mangansulfat-dihydrat auf pH 7.0 eingestellt, auf 1 L mit destilliertem Wasser aufgefüllt und anschließend bei 121 °C für 15 Minuten autoklaviert.

Kultivierungen wurden sowohl in einem klassischen Stahlreaktor mit Rührwerk, als auch in Beutelreaktoren mit Wipp-System unter anoxischen Bedingungen durchgeführt, wobei die Temperatur bei 37 °C und der pH bei 7,0 mit Säure und Lauge (HCl bzw. NaOH) gehalten wurde.

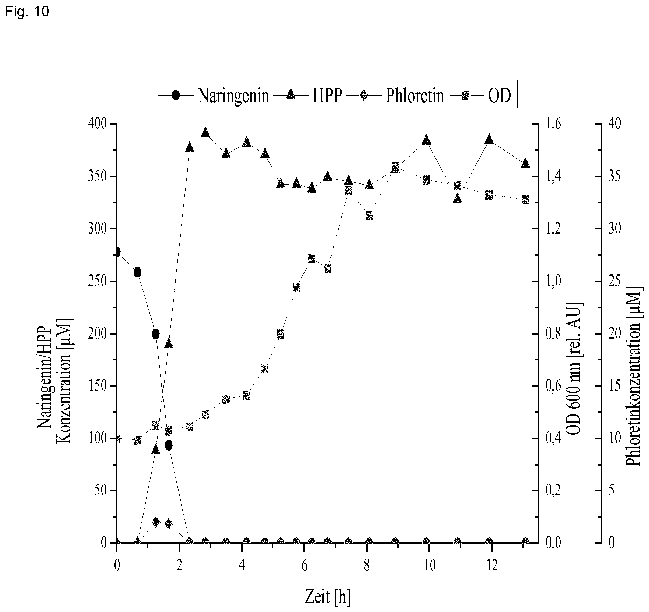

In einer Beispielfermentation wurde dem Medium zu Beginn der Kultivierung 275 µM Naringenin zugegeben und das Wachstum sowie die Umsetzung des Substrates bestimmt (für Ergebnisse siehe Fig. 10).

SEQ ID NO:**1**

SEQ ID NO:**2**

SEQ ID NO:**3**

SEQ ID NO:**4**

SEQ ID NO: 5
codonoptimierte Nukleotidsequenz zur Expression der ERED in *E. coli* BL21, integriert in pET 22b, kloniert wurde mit Nde1 und BamH1:

SEQ ID NO:**6**

SEQ ID NO:**7**

SEQ ID NO:**8**

SEQ ID NO:**9**

## Patentansprüche

1. Transgener Mikroorganismus, enthaltend als Transgen
- einen Nukleinsäure-Abschnitt (a), umfassend oder bestehend aus einem für eine bakterielle Chalconisomerase kodierenden Gen,
und/oder einen Nukleinsäure-Abschnitt (a'), umfassend oder bestehend aus einem für eine pflanzliche Chalconisomerase kodierenden Gen,
sowie
- einen Nukleinsäure-Abschnitt (b), umfassend oder bestehend aus einem für eine bakterielle Enoat-Reduktase kodierenden Gen,
wobei der transgene Mikroorganismus kein Mikroorganismus der Ordnung Clostridiales ist und wobei der Mikroorganismus Chalconisomerase- und Enoat-Reduktase-Aktivität aufweist, jedoch keine Phloretinhydrolase-Aktivität.

2. Mikroorganismus nach Anspruch 1, wobei
das für eine bakterielle Chalconisomerase kodierende Gen für eine Chalconisomerase aus einem Mikroorganismus aus dem Phylus der Firmicutes kodiert,
und/oder
das für eine pflanzliche Chalconisomerase kodierende Gen für eine Chalconisomerase aus *A. thaliana* oder M. *sativa* kodiert,
und/oder
das für eine bakterielle Enoat-Reduktase kodierende Gen für eine Enoat-Reduktase aus einem Mikroorganismus aus dem Phylus der Firmicutes kodiert.

3. Mikroorganismus nach einem der vorangehenden Ansprüche, enthaltend ein oder mehrere identische oder unterschiedliche Vektoren, insbesondere Plasmidvektoren, enthaltend einen
- Nukleinsäure-Abschnitt (a), umfassend oder bestehend aus einem für eine bakterielle Chalconisomerase kodierenden Gen, und/oder einen Nukleinsäure-Abschnitt (a'), umfassend oder bestehend aus einem für eine pflanzliche Chalconisomerase kodierenden Gen,
und/oder
- einen Nukleinsäure-Abschnitt (b), umfassend oder bestehend aus einem für eine bakterielle Enoat-Reduktase kodierenden Gen,
wobei
das für eine bakterielle Chalconisomerase kodierende Gen für eine Chalconisomerase aus einem Mikroorganismus aus dem Phylus der Firmicutes kodiert,
und/oder
das für eine pflanzliche Chalconisomerase kodierende Gen für eine Chalconisomerase aus *A. thaliana* oder M. *sativa* kodiert,
und/oder
das für eine bakterielle Enoat-Reduktase kodierende Gen für eine Enoat-Reduktase aus einem Mikroorganismus aus dem Phylus der Firmicutes kodiert.

4. Mikroorganismus nach Anspruch 3, enthaltend
sowohl ein oder mehrere Vektoren mit einem Nukleinsäure-Abschnitt (a), umfassend oder bestehend aus einem für eine bakterielle Chalconisomerase kodierenden Gen, und/oder einem Nukleinsäure-Abschnitt (a'), umfassend oder bestehend aus einem für eine pflanzliche Chalconisomerase kodierenden Gen,
als auch ein oder mehrere Vektoren mit einem Nukleinsäure-Abschnitt (b), umfassend oder bestehend aus einem für eine bakterielle Enoat-Reduktase kodierenden Gen.

5. Verfahren zur Herstellung eines Dihydrochalkons, unter Verwendung eines transgenen Mikroorganismus, umfassend folgende Schritte:
(i) Bereitstellen eines transgenen Mikroorganismus nach einem der Ansprüche 1 bis 4,
(ii) Hinzufügen von einem oder mehreren Flavanonen und/oder von einer oder mehreren Vorstufen oder einem oder mehreren Derivaten davon zu dem transgenen Mikroorganismus und Kultivieren des transgenen Mikroorganismus unter Bedingungen, die die Umsetzung des bzw. der Flavanone und/oder der Vorstufe(n) oder des Derivats bzw. der Derivate davon zu einem Dihydrochalkon ermöglichen,
(iii) optional: Isolieren sowie gegebenenfalls Aufreinigen des Dihydrochalkons.

6. Verfahren nach Anspruch 5, wobei der transgene Mikroorgansimus ausgewählt ist aus der Gruppe bestehend aus fakultativ anaeroben Mikroorganismen.

7. Verfahren nach Anspruch 5 oder 6, wobei in Schritt (ii) Naringin und/oder ein Aglycon davon hinzugefügt wird bzw. werden.

8. Verfahren nach einem der Ansprüche 5 bis 7, wobei
der Nukleinsäure-Abschnitt (a) eine Nukleotidsequenz gemäß SEQ ID NO:**1** oder eine Nukleotidsequenz mit einer Nukleotidsequenz-Identität von 40 % oder mehr zu SEQ ID NO:**1** umfasst oder daraus besteht
und/oder
der Nukleinsäure-Abschnitt (a') eine Nukleotidsequenz gemäß SEQ ID NO:**6** oder SEQ ID NO:**7** oder eine Nukleotidsequenz mit einer Nukleotidsequenz-Identität von 40 % oder mehr zu SEQ ID NO:**6** oder SEQ ID NO:**7** umfasst oder daraus besteht
und/oder
der Nukleinsäure-Abschnitt (b) eine Nukleotidsequenz gemäß SEQ ID NO:**2** oder SEQ ID NO:**5** oder eine Nukleotidsequenz mit einer Nukleotidsequenz-Identität von 40 % oder mehr zu SEQ ID NO:**2** oder SEQ ID NO:**5** umfasst oder daraus besteht.

9. Verfahren nach einem der Ansprüche 5 bis 8, wobei
die bakterielle Chalconisomerase eine Aminosäuresequenz gemäß SEQ ID NO:**3** oder eine Aminosäuresequenz mit einer Aminosäuresequenz-Identität von 40 % oder mehr zu SEQ ID NO:**3** umfasst bzw. daraus besteht
und/oder
die pflanzliche Chalconisomerase eine Aminosäuresequenz gemäß SEQ ID NO:**8** oder SEQ ID NO:**9** oder eine Aminosäuresequenz mit einer Aminosäuresequenz-Identität von 40 % oder mehr zu SEQ ID NO:**8** oder SEQ ID NO:**9** umfasst bzw. daraus besteht
und/oder
die bakterielle Enoat-Redukatase eine Aminosäuresequenz gemäß SEQ ID NO:**4** oder eine Aminosäuresequenz mit einer Aminosäuresequenz-Identität von 40 % oder mehr zu SEQ ID NO:**4** umfasst bzw. daraus besteht.

## Claims

1. Transgenic microorganism, containing as a transgene
- a nucleid acid portion (a), comprising or consisting of a gene coding for a bacterial chalcone isomerase
and/or a nucleid acid portion (a'), comprising or consisting of a gene coding for a plant-based chalcone isomerase, as well as
- a nucleid acid portion (b), comprising or consisting of a gene coding for a bacterial enoate reductase,
wherein the transgenic microorganism is not a microorganism of the order clostridiales and wherein the microorganism displays chalcone isomerase and enoate reductase activity, but no phloretin hydrolase activity.

2. Microorganism according to claim 1, wherein
the gene coding for a bacterial chalcone isomerase codes for a chalcone isomerase from a microorganism from the phylum of the firmicutes,
and/or
the gene coding for a plant-based chalcone isomerase codes for a chalcone isomerase from *A. thaliana* or M. *sativa,*
and/or
the gene coding for a bacterial enoate reductase codes for an enoate reductase from a microorganism from the phylum of the firmicutes.

3. Microorganism according to any one of the preceding claims, containing one or several identical or different vectors, particularly plasmid vectors, containing a
- nucleid acid portion (a), comprising or consisting of a gene coding for a bacterial chalcone isomerase, and/or a nucleid acid portion (a'), comprising or consisting of a gene coding for a plant-based chalcone isomerase,
and/or
- a nucleid acid portion (b), comprising or consisting of a gene coding for a bacterial enoate reductase,
wherein
the gene coding for a bacterial chalcone isomerase codes for a chalcone isomerase from a microorganism from the phylum of the firmicutes,
and/or
the gene coding for a plant-based chalcone isomerase codes for a chalcone isomerase from *A. thaliana* or *M*. *Sativa,*
and/or
the gene coding for a bacterial enoate reductase codes for an enoate reductase from a microorganism from the phylum of the firmicutes.

4. Microorganism according to claim 3, containing
one or several vectors with a nucleid acid portion (a), comprising or consisting of a gene coding for a bacterial chalcone isomerase, and/or a nucleid acid portion (a'), comprising or consisting of a gene coding for a plant-based chalcone isomerase,
as well as one or several vectors with a nucleic acid portion (b), comprising or consisting of a gene coding for bacterial enoate reductase.

5. Method for producing a dihydrochalcone, with the use of a transgenic microorganism, comprising the following steps:
(i) Providing a transgenic microorganism according to any one of the claims 1 to 4,
(ii) addition of one or several flavanones and/or of one or several precursors or one or several derivatives thereof to the transgenic microorganism and cultivation of the transgenic microorganism under conditions that enable the conversion of the flavanone(s) and/or the precursor(s) or the derivate(s) thereof to a dihydrochalcone,
(iii) optionally: isolation as well as, if applicable, purification of the dihydrochalcone.

6. Method according to claim 5, wherein the transgenic microorganism is selected from the group consisting of facultatively anaerobic microorganisms.

7. Method according to claim 5 or 6, wherein in step (ii) naringin and/or an aglycone thereof is/are added.

8. Method according to any one of the claims 5 to 7, wherein
the nucleic acid portion (a) comprises or consists of a nucleotide sequence according to SEQ ID NO:**1** or a nucleotide sequence with a nucleotide sequence identity of 40% or more with SEQ ID NO:**1**
and/or
the nucleic acid portion (a') comprises or consists of a nucleotide sequence according to SEQ ID NO:**6** or SEQ ID NO:**7** or a nucleotide sequence with a nucleotide sequence identity of 40% or more with SEQ ID NO:**6** or SEQ ID NO:**7**
and/or
the nucleic acid portion (b) comprises or consists of a nucleotide sequence according to SEQ ID NO:**2** or SEQ ID NO:**5** or a nucleotide sequence with a nucleotide sequence identity of 40% or more with SEQ ID NO:**2** or SEQ ID NO:**5**.

9. Method according to any one of the claims 5 to 8, wherein
the bacterial chalcone isomerase comprises or consists of an amino acid sequence according to SEQ ID NO:**3** or an amino acid sequence with an amino acid sequence identity of 40% or more with SEQ ID NO:**3**
and/or
the plant-based chalcone isomerase comprises or consists of an amino acid sequence according to SEQ ID NO:**8** or SEQ ID NO:**9** or an amino acid sequence with an amino acid sequence identity of 40% or more with SEQ ID NO:**8** or SEQ ID NO:**9**
and/or
the bacterial enoate reductase comprises or consists of an amino acid sequence according to SEQ ID NO:**4** or an amino acid sequence with an amino acid sequence identity of 40% or more with SEQ ID NO:**4**.

## Revendications

1. Micro-organisme transgénique contenant, en tant que transgène,
- un segment d'acide nucléique (a) comprenant ou consistant en un gène codant pour une chalcone isomérase bactérienne,
et/ou un segment d'acide nucléique (a') comprenant ou consistant en un gène codant pour une chalcone isomérase végétale,
ainsi qu'
- un segment d'acide nucléique (b) comprenant ou consistant en un gène codant pour une énoate réductase bactérienne,
dans lequel le micro-organisme transgénique n'est pas de micro-organisme de l'ordre Clostridiales et dans lequel le micro-organisme présente de l'activité de chalcone isomérase et d'énoate réductase, mais ne présente aucune activité de phlorétine hydrolase.

2. Micro-organisme selon la revendication 1, dans lequel
le gène codant pour une chalcone isomérase bactérienne code pour une chalcone isomérase à partir d'un micro-organisme du phylum des Firmicutes,
et/ou
le gène codant pour une chalcone isomérase végétale code pour une chalcone isomérase à partir de *A. thaliana* ou M. *sativa,*
et/ou
le gène codant pour une énoate réductase bactérienne code pour une énoate réductase à partir d'un micro-organisme du phylum des Firmicutes.

3. Micro-organisme selon l'une quelconque des revendications précédentes, contenant un ou plusieurs vecteurs identiques ou différents, en particulier vecteurs de plasmide, contenant
- un segment d'acide nucléique (a) comprenant ou consistant en un gène codant pour une chalcone isomérase bactérienne et/ou un segment d'acide nucléique (a') comprenant ou consistant en un gène codant pour une chalcone isomérase végétale,
et/ou
- un segment d'acide nucléique (b) comprenant ou consistant en un gène codant pour une énoate réductase bactérienne,
dans lequel
le gène codant pour une chalcone isomérase bactérienne code pour une chalcone isomérase à partir d'un micro-organisme du phylum des Firmicutes,
et/ou
le gène codant pour une chalcone isomérase végétale code pour une chalcone isomérase à partir de *A. thaliana* ou M. *sativa,*
et/ou
le gène codant pour une énoate réductase bactérienne code pour une énoate réductase à partir d'un micro-organisme du phylum des Firmicutes.

4. Micro-organisme selon la revendication 3, contenant
aussi bien un ou plusieurs vecteurs ayant un segment d'acide nucléique (a) comprenant ou consistant en un gène codant pour une chalcone isomérase bactérienne et/ou un segment d'acide nucléique (a') comprenant ou consistant en un gène codant pour une chalcone isomérase végétale,
qu'un ou plusieurs vecteurs ayant un segment d'acide nucléique (b) comprenant ou consistant en un gène codant pour une énoate réductase bactérienne.

5. Procédé de préparation d'une dihydrochalcone en utilisant un micro-organisme transgénique, comprenant les étapes suivantes :
(i) fournir un micro-organisme transgénique selon l'une quelconque des revendications 1 à 4,
(ii) ajouter au micro-organisme transgénique un ou plusieurs flavanones et/ou un ou plusieurs précurseurs ou un ou plusieurs dérivés de ceux-ci, et cultiver le micro-organisme transgénique dans des conditions qui permettent la transformation du ou bien des flavanones et/ou du (des) précurseur(s) ou du dérivé ou bien des dérivés de ceux-ci, en une dihydrochalcone,
(iii) en option: isoler ainsi que, le cas échéant, purifier la dihydrochalcone.

6. Procédé selon la revendication 5, dans lequel le micro-organisme transgénique est choisi dans le groupe constitué par des micro-organismes anaérobies facultatifs.

7. Procédé selon la revendication 5 ou 6, dans lequel, à l'étape (ii), on ajoute de la naringine et/ou un aglycone de celle-ci.

8. Procédé selon l'une quelconque des revendications 5 à 7, dans lequel
le segment d'acide nucléique (a) comprend ou est constitué par une séquence nucléotidique selon la SEQ ID NO:1 ou une séquence nucléotidique ayant une identité de séquence nucléotidique de 40 %, ou plus, à la SEQ ID NO:1,
et/ou
le segment d'acide nucléique (a') comprend ou est constitué par une séquence nucléotidique selon la SEQ ID NO:6 ou SEQ ID NO:7 ou une séquence nucléotidique ayant une identité de séquence nucléotidique de 40 %, ou plus, à la SEQ ID NO:6 ou SEQ ID NO:7,
et/ou
le segment d'acide nucléique (b) comprend ou est constitué par une séquence nucléotidique selon la SEQ ID NO:2 ou SEQ ID NO:5 ou une séquence nucléotidique ayant une identité de séquence nucléotidique de 40 %, ou plus, à la SEQ ID NO:2 ou SEQ ID NO:5.

9. Procédé selon l'une quelconque des revendications 5 à 8, dans lequel
la chalcone isomérase bactérienne comprend ou bien est constituée par une séquence d'acides aminés selon la SEQ ID NO:3 ou une séquence d'acides aminés ayant une identité de séquence d'acides aminés de 40 %, ou plus, à la SEQ ID NO:3,
et/ou
la chalcone isomérase végétale comprend ou bien est constituée par une séquence d'acides aminés selon la SEQ ID NO:8 ou la SEQ ID NO:9 ou une séquence d'acides aminés ayant une identité de séquence d'acides aminés de 40 %, ou plus, à la SEQ ID NO:8 ou SEQ ID NO:9,
et/ou
l'énoate réductase bactérienne comprend ou bien est constituée par une séquence d'acides aminés selon la SEQ ID NO:4 ou une séquence d'acides aminés ayant une identité de séquence d'acides aminés de 40 %, ou plus, à la SEQ ID NO:4.


### SEQUENCE LISTING

<110> SYMRISE AG
<120> Verfahren zur biotechnologischen Herstellung von Dihydrochalkonen
<140> noch nicht bekannt
   <141> 2013-07-11
<150> DE10 2012 213 492.1
   <151> 2012-07-31
<160> 9
<170> BiSSAP 1.0
<210> 1
   <211> 852
   <212> DNA
   <213> Eubacterium ramulus
<220>
   <221> source
   <222> 1..852
   <223> /mol_type="DNA"
   /organism="Eubacterium ramulus"
<400> 1
   atggcagatt tcaaattcga accaatgaga agtcttatct acgttgactg cgtaagcgaa      60
   gactacagac caaaacttca gagatggatt tataaagtac atattccgga cagcatctct     120
   cagtttgagc cgtatgttac caaatatgca ttttatccgt ccttcccgat tccaccacag     180
   ggtgatcgtt tcggatacgc aagaatgcag ctgacagagc atcactggtt agtaagcgac     240
   cttgatcctc gtcttgagat caaagcaatc gctgagacat tcccgatgga cgtacttgta     300
   tggcagggac agatcccggc agcagctcat acagacgctc agatcgattc tgacggagat     360
   gcaggaaatg cagcccgtaa atccaacaat gcagaaggaa atccatttat ctttgcattc     420
   cttccgatgt ggtgggagaa agacctgaaa ggaaaaggac gtacgatcga ggacggcgca     480
   aactatcgtt tcaatatgac tatcggtttc ccagaaggcg tagacaaagc agagggagag     540
   aaatggttat ttgagaaagt agttccgatt cttcaggcag ctccggagtg tacacgtgta     600
   cttgcaagtg ccgtaaagaa agacatcaac ggatgcgtaa tggattgggt acttgaaatc     660
   tggtttgaga atcagtccgg atggtacaaa gtaatggtag atgacatgaa agcacttgaa     720
   aaaccgtcat gggctcagca ggatgctttc ccgttcctga aaccatacca caatgtttgc     780
   agtgcagcag ttgctgatta tacaccaagc aacaaccttg caaattatcg tggatacatc     840
   accatgagat aa                                                         852
<210> 2
   <211> 2037
   <212> DNA
   <213> Eubacterium ramulus
<220>
   <221> source
   <222> 1..2037
   <223> /mol_type="DNA"
   /organism="Eubacterium ramulus"
<400> 2
   atggcagaaa aaaatcagta ttttccacat ttgtttgagc cgttaaaagt tggttcaaag 60
   acaattaaga accgtattga ggcagcaccg gctttatttg cattcgagca ttatatcgaa 120
   ctgaatccgg atccgtttgg ctataccaca ccggttccgg agcgtgcgtt ccgtatgctg 180
   gaggcaaagg caaaaggagg ggcaggaatt gtatgtctgg gtgagttaag cccgaatcat 240
   gagtatgaca aacggtttcc gtttgaaccg tatcttgatt ttacatccag atcagataag 300
   cagtttgaaa ttatgaagga aactgcggag atgatcaaaa gctatggggc atttccgatg 360
   ggcgagctgc tttcctgcgg tgaaatcaag acaaatatcg gagatggtat caatccgaag 420
   ggaccatctg agaaagatct tccggatggc tctcatgtgg aggcgtttac aaaagaagag 480
   attttaagct gctatcagga ttatgtaact gcatgtaaat ggtttcaggc agcaggctgg 540
   gaaggcatta tgatccactg cggacatggc tggcttccgg cacagttcct gtctccgcaa 600
   tacaataaac gtaccgatga gtatggtgga tcttttgaaa acagagcaag atttactgtt 660
   gatctgttaa aaactgttcg tgaagctatg ggaccggact tcgtgatcga gatccgtgtc 720
   agcagctctg agcatttacc gggcggatta gagctggaag atgctgtaaa ttattgtaaa 780
   ctgtgtgagc catacattga tatgatccat gtctcctgtg gtcattacct gagttcttcc 840
   agaagttggg agttcacaac tgcttatgca ccgcatggtc cgaatattga accggcagct 900
   gttatcaaac agaacgtatc cattccggtt gcggcagtcg gcggcatcaa ttctccggaa 960
   caggcggaag aggcaatcgc gtcaggaaaa atcgatatgg tatccatggg acgtcagttc 1020
   tttgcagatc cggcatttcc aaacaaggca aaagaagggc atgcagatga gatccgtcgc 1080
   tgtctgcgct gcggaagatg ctatccgggt ccgtccggcg agcatgaaac agagatctgg 1140
   acggtgaaat tcccaccact ggattcctgt accatcaatc catatgatgt atggccggca 1200
   tctcatcata aagtccttcc ggaccgcatg ccgaaaccgg aagcaagccg taaggtattg 1260
   gtagtaggcg gcggctgtgg cggtctgcag acagcgatca cagcatcaga cagaggtcat 1320
   caggtaatcc tgtgcgaaaa atccggagta ttaggcggtc tgatcaattt tacggatcat 1380
   acggatcata aagtagatat cagaaacttc aaagatctgc tgatccgcga tgtggagaaa 1440
   cgtccgatcg aagtaagatt aaactgtgaa gtaacaccgg aactcatcag agaaattgct 1500
   ccggaagcag ttgtactggc cgtcggatcc gatgatctga tccttccaat cgagggaatt 1560
   gaaaatgcgg taacagcaat ggacgtatac agcaatgact ttgcaggtct tggaaagagc 1620
   accatcgtac tcggtggcgg tctggttggc tgtgaggcag ccgcagatta tattgatcac 1680
   ggtgtagaga caacgattgt tgaaatgaaa ggtgcgctga tgccggagac aaccggtctg    1740
   taccgtacag ctgtacatga tttcatcgac aaaaacggcg gcaaatacga agtaaatgca    1800
   aaagttgtca aagttggcaa agattttgtg gtagcggaac aagatgggaa agagattacc    1860
   atcaaagcag attctgttgt caatgcaatg ggacgccgtg cgcatgcgac agaagcactt    1920
   gagacagcta tcaaagaagc tggtattccg gtatggaaga tcggtgactg tgtccgtgcg    1980
   cgtcagatcg gtgatgcggt aagagaaggc tggaccgcag caatggaaat tatctaa       2037
<210> 3
   <211> 678
   <212> PRT
   <213> Eubacterium ramulus
<220>
   <221> SOURCE
   <222> 1..678
   <223> /mol_type="protein"
   /organism="Eubacterium ramulus"
<400> 3
   Met Ala Glu Lys Asn Gln Tyr Phe Pro His Leu Phe Glu Pro Leu Lys
   1               5                   10                  15
   Val Gly Ser Lys Thr Ile Lys Asn Arg Ile Glu Ala Ala Pro Ala Leu
               20                  25                  30
   Phe Ala Phe Glu His Tyr Ile Glu Leu Asn Pro Asp Pro Phe Gly Tyr
           35                  40                  45
   Thr Thr Pro Val Pro Glu Arg Ala Phe Arg Met Leu Glu Ala Lys Ala
       50                  55                  60
   Lys Gly Gly Ala Gly Ile Val Cys Leu Gly Glu Leu Ser Pro Asn His
   65                  70                  75                  80
   Glu Tyr Asp Lys Arg Phe Pro Phe Glu Pro Tyr Leu Asp Phe Thr Ser
                   85                  90                  95
   Arg Ser Asp Lys Gln Phe Glu Ile Met Lys Glu Thr Ala Glu Met Ile
               100                 105                 110
   Lys Ser Tyr Gly Ala Phe Pro Met Gly Glu Leu Leu Ser Cys Gly Glu
           115                 120                 125
   Ile Lys Thr Asn Ile Gly Asp Gly Ile Asn Pro Lys Gly Pro Ser Glu
       130                 135                 140
   Lys Asp Leu Pro Asp Gly Ser His Val Glu Ala Phe Thr Lys Glu Glu
   145                 150                 155                 160
   Ile Leu Ser Cys Tyr Gln Asp Tyr Val Thr Ala Cys Lys Trp Phe Gln
                   165                 170                 175
   Ala Ala Gly Trp Glu Gly Ile Met Ile His Cys Gly His Gly Trp Leu
               180                 185                 190
   Pro Ala Gln Phe Leu Ser Pro Gln Tyr Asn Lys Arg Thr Asp Glu Tyr
           195                 200                 205
   Gly Gly Ser Phe Glu Asn Arg Ala Arg Phe Thr Val Asp Leu Leu Lys
       210                 215                 220
   Thr Val Arg Glu Ala Met Gly Pro Asp Phe Val Ile Glu Ile Arg Val
   225                 230                 235                 240
   Ser Ser Ser Glu His Leu Pro Gly Gly Leu Glu Leu Glu Asp Ala Val
                   245                 250                 255
   Asn Tyr Cys Lys Leu Cys Glu Pro Tyr Ile Asp Met Ile His Val Ser
               260                 265                 270
   Cys Gly His Tyr Leu Ser Ser Ser Arg Ser Trp Glu Phe Thr Thr Ala
           275                 280                 285
   Tyr Ala Pro His Gly Pro Asn Ile Glu Pro Ala Ala Val Ile Lys Gln
           290                 295                 300
   Asn Val Ser Ile Pro Val Ala Ala Val Gly Gly Ile Asn Ser Pro Glu
   305                 310                 315                 320
   Gln Ala Glu Glu Ala Ile Ala Ser Gly Lys Ile Asp Met Val Ser Met
                   325                 330                 335
   Gly Arg Gln Phe Phe Ala Asp Pro Ala Phe Pro Asn Lys Ala Lys Glu
               340                 345                 350
   Gly His Ala Asp Glu Ile Arg Arg Cys Leu Arg Cys Gly Arg Cys Tyr
           355                 360                 365
   Pro Gly Pro Ser Gly Glu His Glu Thr Glu Ile Trp Thr Val Lys Phe
       370                 375                 380
   Pro Pro Leu Asp Ser Cys Thr Ile Asn Pro Tyr Asp Val Trp Pro Ala
   385                 390                 395                 400
   Ser His His Lys Val Leu Pro Asp Arg Met Pro Lys Pro Glu Ala Ser
                   405                 410                 415
   Arg Lys Val Leu Val Val Gly Gly Gly Cys Gly Gly Leu Gln Thr Ala
               420                 425                 430
   Ile Thr Ala Ser Asp Arg Gly His Gln Val Ile Leu Cys Glu Lys Ser
           435                 440                 445
   Gly Val Leu Gly Gly Leu Ile Asn Phe Thr Asp His Thr Asp His Lys
       450                 455                 460
   Val Asp Ile Arg Asn Phe Lys Asp Leu Leu Ile Arg Asp Val Glu Lys
   465                 470                 475                 480
   Arg Pro Ile Glu Val Arg Leu Asn Cys Glu Val Thr Pro Glu Leu Ile
                   485                 490                 495
   Arg Glu Ile Ala Pro Glu Ala Val Val Leu Ala Val Gly Ser Asp Asp
               500                 505                 510
   Leu Ile Leu Pro Ile Glu Gly Ile Glu Asn Ala Val Thr Ala Met Asp
           515                 520                 525
   Val Tyr Ser Asn Asp Phe Ala Gly Leu Gly Lys Ser Thr Ile Val Leu
       530                 535                 540
   Gly Gly Gly Leu Val Gly Cys Glu Ala Ala Ala Asp Tyr Ile Asp His
   545                 550                 555                 560
   Gly Val Glu Thr Thr Ile Val Glu Met Lys Gly Ala Leu Met Pro Glu
                   565                 570                 575
   Thr Thr Gly Leu Tyr Arg Thr Ala Val His Asp Phe Ile Asp Lys Asn
               580                 585                 590
   Gly Gly Lys Tyr Glu Val Asn Ala Lys Val Val Lys Val Gly Lys Asp
           595                 600                 605
   Phe Val Val Ala Glu Gln Asp Gly Lys Glu Ile Thr Ile Lys Ala Asp
       610                 615                 620
   Ser Val Val Asn Ala Met Gly Arg Arg Ala His Ala Thr Glu Ala Leu
   625                 630                 635                 640
   Glu Thr Ala Ile Lys Glu Ala Gly Ile Pro Val Trp Lys Ile Gly Asp
                   645                 650                 655
   Cys Val Arg Ala Arg Gln Ile Gly Asp Ala Val Arg Glu Gly Trp Thr
               660                 665                 670
   Ala Ala Met Glu Ile Ile
           675
<210> 4
   <211> 283
   <212> PRT
   <213> Eubacterium ramulus
<220>
   <221> SOURCE
   <222> 1..283
   <223> /mol_type="protein"
   /organism="Eubacterium ramulus"
<400> 4
   Met Ala Asp Phe Lys Phe Glu Pro Met Arg Ser Leu Ile Tyr Val Asp
   1               5                   10                  15
   Cys Val Ser Glu Asp Tyr Arg Pro Lys Leu Gln Arg Trp Ile Tyr Lys
               20                  25                  30
   Val His Ile Pro Asp Ser Ile Ser Gln Phe Glu Pro Tyr Val Thr Lys
           35                  40                  45
   Tyr Ala Phe Tyr Pro Ser Phe Pro Ile Pro Pro Gln Gly Asp Arg Phe
       50                  55                  60
   Gly Tyr Ala Arg Met Gln Leu Thr Glu His His Trp Leu Val Ser Asp
   65                  70                  75                  80
   Leu Asp Pro Arg Leu Glu Ile Lys Ala Ile Ala Glu Thr Phe Pro Met
                   85                  90                  95
   Asp Val Leu Val Trp Gln Gly Gln Ile Pro Ala Ala Ala His Thr Asp
               100                 105                 110
   Ala Gln Ile Asp Ser Asp Gly Asp Ala Gly Asn Ala Ala Arg Lys Ser
           115                 120                 125
   Asn Asn Ala Glu Gly Asn Pro Phe Ile Phe Ala Phe Leu Pro Met Trp
       130                 135                 140
   Trp Glu Lys Asp Leu Lys Gly Lys Gly Arg Thr Ile Glu Asp Gly Ala
   145                 150                 155                 160
   Asn Tyr Arg Phe Asn Met Thr Ile Gly Phe Pro Glu Gly Val Asp Lys
                   165                 170                 175
   Ala Glu Gly Glu Lys Trp Leu Phe Glu Lys Val Val Pro Ile Leu Gln
               180                 185                 190
   Ala Ala Pro Glu Cys Thr Arg Val Leu Ala Ser Ala Val Lys Lys Asp
           195                 200                 205
   Ile Asn Gly Cys Val Met Asp Trp Val Leu Glu Ile Trp Phe Glu Asn
       210                 215                 220
   Gln Ser Gly Trp Tyr Lys Val Met Val Asp Asp Met Lys Ala Leu Glu
   225                 230                 235                 240
   Lys Pro Ser Trp Ala Gln Gln Asp Ala Phe Pro Phe Leu Lys Pro Tyr
                   245                 250                 255
   His Asn Val Cys Ser Ala Ala Val Ala Asp Tyr Thr Pro Ser Asn Asn
               260                 265                 270
   Leu Ala Asn Tyr Arg Gly Tyr Ile Thr Met Arg
           275                 280
<210> 5
   <211> 2037
   <212> DNA
   <213> Eubacterium ramulus
<220>
   <221> source
   <222> 1..2037
   <223> /mol_type="DNA"
   /organism="Eubacterium ramulus"
<400> 5
   atggcagaaa agaaccaata cttcccgcac ctgtttgaac cgctgaaagt cggctctaaa 60
   accattaaaa atcgcatcga agcagcaccg gccctgtttg cattcgaaca ttatatcgaa 120
   ctgaacccgg acccgtttgg ttacaccacg ccggtgccgg aacgtgcatt ccgtatgctg 180
   gaagccaaag caaaaggcgg tgccggcatt gtttgtctgg gtgaactgag cccgaatcac 240
   gaatatgata aacgctttcc gttcgaaccg tacctggatt ttaccagccg ttctgacaaa 300
   cagttcgaaa ttatgaaaga aacggcagaa atgatcaaaa gctatggcgc ttttccgatg 360
   ggtgaactgc tgtcgtgcgg tgaaatcaaa accaacattg gcgatggtat caatccgaaa 420
   ggcccgtcag aaaaagatct gccggacggt tcgcatgtgg aagccttcac caaagaagaa 480
   atcctgtcat gttaccagga ttacgttacg gcatgcaaat ggttccaagc ggccggctgg 540
   gaaggtatta tgatccattg tggccacggt tggctgccgg cgcagtttct gagcccgcaa 600
   tataacaaac gcaccgatga atacggcggt tcttttgaaa atcgtgcgcg cttcaccgtc 660
   gatctgctga aaacggtgcg tgaagcgatg ggcccggact tcgtgattga aatccgtgtt 720
   agctctagtg aacatctgcc gggcggtctg gaactggaag atgcggtgaa ctattgcaaa 780
   ctgtgtgaac cgtacattga catgatccat gttagttgcg gccactatct gtcctcatcg 840
   cgctcctggg aatttaccac ggcttacgcg ccgcacggtc cgaacatcga accggcagct 900
   gtcattaaac agaatgtgag catcccggtt gcagcagtcg gcggtatcaa ctctccggaa 960
   caagcggaag aagccattgc aagtggcaaa atcgatatgg ttagcatggg ccgtcagttt 1020
   ttcgctgacc cggcgtttcc gaataaagca aaagaaggcc atgctgatga aattcgtcgc 1080
   tgcctgcgtt gtggtcgctg ctatccgggc ccgagtggtg aacacgaaac cgaaatctgg 1140
   acggtgaaat tcccgccgct ggatagttgt accattaacc cgtacgacgt gtggccggca 1200
   tcccatcaca aagttctgcc ggatcgcatg ccgaaaccgg aagcgtcccg taaagttctg 1260
   gtggttggcg gtggctgtgg tggtctgcag accgcaatca cggcatcaga ccgcggccat 1320
   caagtcattc tgtgcgaaaa atcgggtgtg ctgggtggcc tgattaactt taccgatcat 1380
   acggaccaca aagttgatat tcgcaatttc aaagatctgc tgatccgtga cgtcgaaaaa 1440
   cgcccgattg aagttcgtct gaattgtgaa gtcaccccgg aactgattcg tgaaatcgct 1500
   ccggaagcag tcgtgctggc agtgggcagt gatgacctga ttctgccgat cgaaggtatt 1560
   gaaaacgccg ttaccgcaat ggatgtctat agcaatgact ttgccggcct gggtaaatct 1620
   acgatcgtgc tgggtggcgg tctggttggc tgcgaagcag ctgcggatta tatcgatcat 1680
   ggcgtggaaa ccacgattgt tgaaatgaaa ggcgcactga tgccggaaac cacgggtctg 1740
   tatcgtaccg ctgtgcacga ttttattgac aaaaacggcg gtaaatacga agtcaatgcc 1800
   aaagttgtca aagtgggcaa agatttcgtg gttgcagaac aggacggtaa agaaattacc 1860
   atcaaagcgg attctgtcgt gaatgcgatg ggccgtcgcg ctcacgcaac cgaagctctg 1920
   gaaacggcga ttaaagaagc cggcatcccg gtttggaaaa ttggtgattg cgtccgtgcc 1980
   cgccaaatcg gtgacgcagt tcgtgaaggc tggacggctg caatggaaat catctaa 2037
<210> 6
   <211> 897
   <212> DNA
   <213> Medicago sativa
<220>
   <221> source
   <222> 1..897
   <223> /mol_type="DNA" /organism="Medicago sativa"
<400> 6
   atggctgcat caatcaccgc aatcactgtg gagaaccttg aatacccagc ggtggttacc      60
   tctccggtca ccggcaaatc atatttcctc ggtggcgctg gggagagagg attgaccatt     120
   gaaggaaact tcatcaagtt cactgccata ggtgtttatt tggaagatat agcagtggct     180
   tcactagctg ccaaatggaa gggtaaatca tctgaagagt tacttgagac ccttgacttt     240
   tacagagaca tcatctcagg tccctttgaa aagttaatta gagggtcaaa gattagggaa     300
   ttgagtggtc ctgagtactc aaggaaggtt atggagaact gtgtggcaca cttgaaatca     360
   gttggaactt atggagatgc agaagctgaa gctatgcaaa aatttgctga agctttcaag     420
   cctgttaatt ttccacctgg tgcctctgtt ttctacaggc aatcacctga tggaatatta     480
   gggcttagtt tctctccgga tacaagtata ccagaaaagg aggctgcact catagagaac     540
   aaggcagttt catcagcagt gttggagact atgatcggcg agcacgctgt ttcccctgat     600
   cttaagcgct gtttagctgc aagattacct gcgttgttga acgagggtgc tttcaagatt     660
   ggaaactgat gatgattata ctcctatatc actgcatttc caaaagcgtt gcagcacaag     720
   aatgagacca tgaacttttt taagtctaca cgtttaattt tttgtatatc tatttacctt     780
   cttattagta tcaataatat gaaatgaaag atcttgcttt ctactcttgt actatttctg     840
   tgatagataa tgttaatgag tatcttcatc aataaaagtg atttgttttg tttgttc        897
<210> 7
   <211> 765
   <212> DNA
   <213> Arabidopsis thaliana
<220>
   <221> source
   <222> 1..765
   <223> /mol_type="DNA"
   /organism="Arabidopsis thaliana"
<400> 7
   atgtcttcat ccaacgcctg cgcctctccg tcaccgttcc ccgccgtcac gaagcttcat      60
   gtagactccg tcacgtttgt accgtccgtc aagtcaccgg cctcctccaa tccattattc     120
   ctcggcggcg ccggtgtccg aggccttgat atccaaggta aattcgtgat cttcaccgtc     180
   attggagtat acctagaggg taacgccgtt ccttctctat ctgtcaagtg gaagggaaaa     240
   actacggagg agctaacaga atctatcccg ttcttccgtg aaatagtcac cggtgcgttt     300
   gagaagttta tcaaggtgac aatgaaactg ccgttaacgg gacaacaata ttcggagaaa     360
   gtgacggaga attgtgtggc tatatggaaa caattagggc tttatacgga ctgtgaagct     420
   aaagctgtgg agaagttctt ggagatcttc aaggaagaaa cattccctcc cggttcatcg     480
   atcctcttcg ctctctcccc taccggctct cttacggttg cgttttcgaa agatgatagt     540
   atccctgaaa ccgggatcgc tgtgatcgag aacaaattgt tggcggaggc ggttctggaa     600
   tctatcatcg ggaagaacgg tgtgtcacct ggcactaggt taagtgttgc agaaagatta     660
   tctcagctaa tgatgaagaa caaggacgaa aaggaagtta gtgatcactc tgttgaggaa     720
   aaactagcca aagagaactg agaatgatag atttttcttg tgttt                     765
<210> 8
   <211> 222
   <212> PRT
   <213> Medicago sativa
<220>
   <221> SOURCE
   <222> 1..222
   <223> /mol_type="protein"
   /organism="Medicago sativa"
<400> 8
   Met Ala Ala Ser Ile Thr Ala Ile Thr Val Glu Asn Leu Glu Tyr Pro
   1               5                   10                  15
   Ala Val Val Thr Ser Pro Val Thr Gly Lys Ser Tyr Phe Leu Gly Gly
               20                  25                  30
   Ala Gly Glu Arg Gly Leu Thr Ile Glu Gly Asn Phe Ile Lys Phe Thr
           35                  40                  45
   Ala Ile Gly Val Tyr Leu Glu Asp Ile Ala Val Ala Ser Leu Ala Ala
       50                  55                  60
   Lys Trp Lys Gly Lys Ser Ser Glu Glu Leu Leu Glu Thr Leu Asp Phe
   65                  70                  75                  80
   Tyr Arg Asp Ile Ile Ser Gly Pro Phe Glu Lys Leu Ile Arg Gly Ser
                   85                  90                  95
   Lys Ile Arg Glu Leu Ser Gly Pro Glu Tyr Ser Arg Lys Val Met Glu
               100                 105                 110
   Asn Cys Val Ala His Leu Lys Ser Val Gly Thr Tyr Gly Asp Ala Glu
           115                 120                 125
   Ala Glu Ala Met Gln Lys Phe Ala Glu Ala Phe Lys Pro Val Asn Phe
       130                 135                 140
   Pro Pro Gly Ala Ser Val Phe Tyr Arg Gln Ser Pro Asp Gly Ile Leu
   145                 150                 155                 160
   Gly Leu Ser Phe Ser Pro Asp Thr Ser Ile Pro Glu Lys Glu Ala Ala
                   165                 170                 175
   Leu Ile Glu Asn Lys Ala Val Ser Ser Ala Val Leu Glu Thr Met Ile
               180                 185                 190
   Gly Glu His Ala Val Ser Pro Asp Leu Lys Arg Cys Leu Ala Ala Arg
           195                 200                 205
   Leu Pro Ala Leu Leu Asn Glu Gly Ala Phe Lys Ile Gly Asn
       210                 215                 220
<210> 9
   <211> 246
   <212> PRT
   <213> Arabidopsis thaliana
<220>
   <221> SOURCE
   <222> 1..246
   <223> /mol_type="protein"
   /organism="Arabidopsis thaliana"
<400> 9
   Met Ser Ser Ser Asn Ala Cys Ala Ser Pro Ser Pro Phe Pro Ala Val
   1               5                   10                  15
   Thr Lys Leu His Val Asp Ser Val Thr Phe Val Pro Ser Val Lys Ser
               20                  25                  30
   Pro Ala Ser Ser Asn Pro Leu Phe Leu Gly Gly Ala Gly Val Arg Gly
           35                  40                  45
   Leu Asp Ile Gln Gly Lys Phe Val Ile Phe Thr Val Ile Gly Val Tyr
       50                  55                  60
   Leu Glu Gly Asn Ala Val Pro Ser Leu Ser Val Lys Trp Lys Gly Lys
   65                  70                  75                  80
   Thr Thr Glu Glu Leu Thr Glu Ser Ile Pro Phe Phe Arg Glu Ile Val
                   85                  90                  95
   Thr Gly Ala Phe Glu Lys Phe Ile Lys Val Thr Met Lys Leu Pro Leu
               100                 105                 110
   Thr Gly Gln Gln Tyr Ser Glu Lys Val Thr Glu Asn Cys Val Ala Ile
           115                 120                 125
   Trp Lys Gln Leu Gly Leu Tyr Thr Asp Cys Glu Ala Lys Ala Val Glu
       130                 135                 140
   Lys Phe Leu Glu Ile Phe Lys Glu Glu Thr Phe Pro Pro Gly Ser Ser
   145                 150                 155                 160
   Ile Leu Phe Ala Leu Ser Pro Thr Gly Ser Leu Thr Val Ala Phe Ser
                   165                 170                 175
   Lys Asp Asp Ser Ile Pro Glu Thr Gly Ile Ala Val Ile Glu Asn Lys
               180                 185                 190
   Leu Leu Ala Glu Ala Val Leu Glu Ser Ile Ile Gly Lys Asn Gly Val
           195                 200                 205
   Ser Pro Gly Thr Arg Leu Ser Val Ala Glu Arg Leu Ser Gln Leu Met
       210                 215                 220
   Met Lys Asn Lys Asp Glu Lys Glu Val Ser Asp His Ser Val Glu Glu
   225                 230                 235                 240
   Lys Leu Ala Lys Glu Asn
                   245